# EUROPEAN PATENT APPLICATION

(11) **EP 4 386 008 A1**
(43) Date of publication of application: **19.06.2024**
(21) Application number: 22855400.2
(22) Date of filing: 08.08.2022
(51) Int. Cl.: C07K 19/00, A61K 38/16, A61P 35/00

(54) **NEW MUTANT OF RECOMBINANT GANODERMA LUCIDUM IMMUNOREGULATORY PROTEIN AND APPLICATION THEREOF**

(30) Priority: 09.08.2021 CN 202110908697
(71) Applicant: Changchun Intellicrown Pharmaceutical Co., Ltd., Changchun, Jilin 130507 (CN); Shanghai Intellicrown Biotechnology Co., Ltd., Shanghai 201318 (CN)
(72) Inventor: ZHANG, Xin, Changchun, Jilin 130507 (CN); SONG, Gongming, Changchun, Jilin 130507 (CN); JIA, Wei, Changchun, Jilin 130507 (CN); ZHANG, Xitian, Changchun, Jilin 130507 (CN)
(74) Representative: Weickmann & Weickmann PartmbB
(86) International application number: PCT/CN2022/110916
(87) International publication number: WO 2023/016419

(57) **Abstract**

The present application relates to a recombinant protein comprising a spacer peptide and a recombinant *Ganoderma lucidum* immunomodulatory protein mutant (rLZ-8 mutant), wherein an amino acid sequence of the rLZ-8 mutant comprises at least one amino acid mutation as compared with an amino acid sequence as shown in SEQ ID NO: 10. The present application further relates to use of the recombinant protein for treating diseases.

## Description

### Field of the Invention

The present application relates to the field of biomedicine, in particular, to a recombinant *Ganoderma lucidum* immunomodulatory protein mutant (rLZ-8 mutant), and its use in the treatment of a tumor.

### Background of the Invention

*Ganoderma lucidum* immunomodulatory protein (LZ-8) was isolated and purified from *Ganoderma lucidum* mycelium extract by Kino, *et al.* in 1989. The extracted LZ-8 comprises 1.3% polysaccharide, which has a mitogenic activity *in vitro,* and an immunomodulatory activity *in vivo.* At the same time, the LZ-8 has an agglutinating effect on sheep erythrocytes, but not on human erythrocytes (Types A, B, AB and O). *In vivo* studies have found that the LZ-8 prevented the occurrence of systemic allergic reaction in mice if repeatedly administered.

The recombinant *Ganoderma lucidum* immunomodulatory protein (rLZ-8) is obtained by gene recombination technique. Compared with a naturally occurring *Ganoderma lucidum* immunomodulatory protein, it has the same amino acid sequence and similar activity, but does not contain any polysaccharide. Studies have shown that rLZ-8 can not only rapidly and effectively induce apoptosis of a plurality of tumor cells, but also effectively kill tumor cells while maintaining or increasing the level of white blood cells in an *in vivo* mouse tumor model. In addition, the use of rLZ-8 for treating tissue fibrosis, focal cerebral ischemia, thrombocytopenia, osteoporosis and heart failure and the like has also been published and granted.

The recombinant *Ganoderma lucidum* immunomodulatory protein mutant (rLZ-8 mutant) is obtained by mutating one or several amino acids of rLZ-8. The mechanism studies have found that the rLZ-8 mutant can bind to the epidermal growth factor receptor (EGFR) on the surface of human cells for internalization into a cell, and be remained within the cell due to non-degradability. Then, it blocks the cell membrane circulation via the high-intensity internalization, and finally results in cell shrinkage, rupture, and death. The results of tumor cell killing test have shown that the rLZ-8 mutant has a better tumor killing effect than rLZ-8. The results of Biacore study have shown that the rLZ-8 mutant has a better affinity with the EGFR; and the experimental results of orthotopic transplantation tumor model mice of a plurality of human cancer cells have shown that the rLZ-8 mutant is superior to the rLZ-8 in terms of prolonging the survival and inhibiting the tumor growth in mice.

However, even though the rLZ-8 and the rLZ-8 mutant exhibit tumor killing effects, both of them face a challenge of low protein expression level and difficulty to industrialization. On the other hand, it is also a focus to be considered during the drug development if the protein molecules are safe for human beings.

### Summary of the Invention

The present application provides a recombinant protein comprising a spacer peptide and a recombinant *Ganoderma lucidum* immunomodulatory protein mutant (rLZ-8 mutant), wherein compared with the amino acid sequence as shown in SEQ ID NO: 10, the amino acid sequence of the rLZ-8 mutant comprises at least one amino acid mutation. The recombinant protein of the present application has at least one characteristic selected from the group consisting of: (1) high expression quantity and/or secretion quantity (e.g., in a yeast expression system); (2) high safety, meeting the safety requirements of finished medicines, e.g., passing the pre-clinical safety assessment, and/or obtaining the clinical trial license; (3) easiness of entering into a cell through internalization; and (4) inhibition of tumor growth and strong ability to kill tumor cells.

In an aspect, the present application provides a recombinant protein comprising a spacer peptide and a recombinant *Ganoderma lucidum* immunomodulatory protein mutant (rLZ-8 mutant), wherein compared with the amino acid sequence as shown in SEQ ID NO: 10, said amino acid sequence of said rLZ-8 mutant comprises at least one amino acid mutation.

In some embodiments, said rLZ-8 mutant comprises an amino acid sequence as shown in any one of SEQ ID NOS: 13 and 16-21.

In some embodiments, said rLZ-8 mutant comprises an amino acid sequence as shown in SEQ ID NO: 13.

In some embodiments, said spacer peptide comprises 1-5 EAs.

In some embodiments, said spacer peptide comprises 2 EAs.

In some embodiments, said spacer peptide comprises an amino acid sequence as shown in any one of SEQ ID NOS: 8-9.

In some embodiments, said spacer peptide comprises an amino acid sequence as shown in SEQ ID NO: 8.

In some embodiments, said spacer peptide is located at an N-terminus of said rLZ-8 or a mutant thereof.

In some embodiments, said recombinant protein comprises an amino acid sequence as shown in any one of SEQ ID NOS: 14, 15, and 22-33.

In some embodiments, said recombinant protein comprises an amino acid sequence as shown in SEQ ID NO: 14.

In another aspect, the present application provides an isolated nucleic acid molecule encoding said recombinant protein.

In another aspect, the present application provides a vector comprising said isolated nucleic acid molecule.

In another aspect, the present application provides a cell comprising said isolated nucleic acid molecule and/or said vector.

In some embodiments, said cell is a eukaryocyte.

In some embodiments, said cell is a yeast cell.

In another aspect, the present application provides a polypeptide comprising said recombinant protein.

In another aspect, the present application provides a method of preparing said recombinant protein comprising culturing said cell under conditions that allow to express said recombinant protein.

In another aspect, the present application provides a pharmaceutical composition comprising said recombinant protein, said isolated nucleic acid molecule, said vector, said polypeptide and/or the cell, and optionally a pharmaceutically acceptable carrier.

In another aspect, the present application provides a kit comprising said recombinant protein, said isolated nucleic acid molecule, said vector, said cell, said polypeptide and/or said pharmaceutical composition.

In another aspect, the present application provides a drug delivery device comprising said recombinant protein, said isolated nucleic acid molecule, said vector, said cell, said polypeptide and/or said pharmaceutical composition.

In another aspect, the present application provides a method of preventing, alleviating or treating a tumor comprising administering to a subject in need thereof said recombinant protein, said isolated nucleic acid molecule, said vector, said cell, said polypeptide and/or said pharmaceutical composition.

In another aspect, the present application provides use of said recombinant protein, said isolated nucleic acid molecule, said vector, said cell, said polypeptide and/or said pharmaceutical composition in the manufacture of a drug for treating, alleviating or treating a tumor.

In another aspect, the present application provides said recombinant protein, said isolated nucleic acid molecule, said vector, said cell, said polypeptide and/or said pharmaceutical composition for preventing, alleviating or treating a tumor.

In some embodiments, said tumor comprises a non-solid tumor.

In some embodiments, said tumor comprises a solid tumor.

In some embodiments, said tumor comprises a tumor having EGFR expression and/or abnormal EGFR expression.

In some embodiments, said tumor comprises a tumor associated with an EGFR mutation. In some embodiments, said tumor comprises a tumor associated with a BRAF mutation. In some embodiments, said tumor comprises a tumor associated with a KRAS mutation. In some embodiments, said tumor comprises a tumor associated with an HER2 mutation.

In some embodiments, said tumor comprises colorectal cancer, lung cancer, liver cancer, breast cancer, gastric cancer, renal cancer, bladder cancer, neuroblastoma, ovarian cancer, epithelial squamous carcinoma, and/or pancreatic cancer. Persons skilled in the art can readily recognize other aspects and advantages of the present application form the detailed description below. The following detailed description only shows and describes exemplary embodiments of the present application. As persons skilled in the art will appreciate, the present application enables persons skilled in the art to make modifications to the disclosed embodiments without departing the spirit and scope of the invention involved in the present application. Correspondingly, the accompany drawings and description in the specification of the present application are only illustrative, rather than restrictive.

### Brief Description of the Drawings

The specific features of the invention involved in the present application are shown in the appended claims. By referring to the exemplary embodiments as detailed below and the accompanying drawings, the features and advantages of the invention involved in the present application can be better understood. The accompany drawings are briefly described as follows:
FIG. 1 shows the results of a plurality of yeast strains expressing an rLZ-8 (SEQ ID NO: 10), wherein M: marker; S: EAEA+rLZ-8 mutant (SEQ ID NO: 14) working reference (1 mg/ml); 1-11: rLZ-8 (SEQ ID NO: 10) Strains 1-11.
FIG. 2 shows the results of a plurality of yeast strains expressing an rLZ-8 mutant (SEQ ID NO: 13), wherein M: marker; S: EAEA+rLZ-8 mutant (SEQ ID NO: 14) working reference (1 mg/ml); 1-11: rLZ-8 mutant (SEQ ID NO: 13) Strains 1-11.
FIG. 3 shows the results of a plurality of yeast strains expressing a recombinant protein of EAEA+rLZ-8 (SEQ ID NO: 11), wherein M: marker; S: rLZ-8 (SEQ ID NO: 10) working reference (0.5 mg/ml); 1-6: EAEA+rLZ-8 (SEQ ID NO: 11) Strains 1-6.
FIG. 4 shows the results of a plurality of yeast strains expressing a recombinant protein of EAEA+rLZ-8 mutant (SEQ ID NO: 14), wherein M: marker; S: rLZ-8 (SEQ ID NO: 10) standard (250 mg/L); 1-6: EAEA+rLZ-8 mutant (SEQ ID NO: 14) Strains 1-6.
FIG. 5 shows the results of a plurality of yeast strains expressing a recombinant protein of EEAEAEAEPK+rLZ-8 (SEQ ID NO: 12), wherein M: marker; S: EAEA+rLZ-8 mutant (SEQ ID NO: 14) working reference (1 mg/ml); 1-11: EEAEAEAEPK+rLZ-8 (SEQ ID NO: 12) Strains 1-11.
FIG. 6 shows the results of a plurality of yeast strains expressing a recombinant protein of EEAEAEAEPK+rLZ-8 mutant (SEQ ID NO: 15), wherein M: marker; S: EAEA+rLZ-8 mutant (SEQ ID NO: 14) working reference (1 mg/ml); 1-11: rLZ-8 mutant (SEQ ID NO: 15) Strains 1-11.
FIG. 7 shows the comparison of expression results of recombinant proteins of rLZ-8 or a mutant thereof linked via different spacer peptides, in which, M: marker; S: EAEA+rLZ-8 mutant (SEQ ID NO: 14) working reference (1mg/ml); 1: rLZ-8 mutant (SEQ ID NO: 13); 2: EAEA+ rLZ-8 mutant (SEQ ID NO: 14); 3: EEAEAEAEPK+ rLZ-8 mutant (SEQ ID NO: 15); 4: rLZ-8 (SEQ ID NO: 10); 5: EAEA+ rLZ-8 (SEQ ID NO: 11); 6: EEAEAEAEPK+ rLZ-8 (SEQ ID NO: 12).
FIG. 8 shows a dose-response curve of a recombinant protein as shown in SEQ ID NO: 14 acting on CR20035B organoid system.
FIG. 9 shows a dose-response curve of a recombinant protein as shown in SEQ ID NO: 14 acting on a CR5043B organoid system.
FIG. 10 shows a dose-response curve of a recombinant protein as shown in SEQ ID NO: 14 acting on a CR5082B organoid system.
FIG. 11 shows a dose-response curve of a recombinant protein as shown in SEQ ID NO: 14 acting on a CR3099B organoid system.
FIG. 12 shows a dose-response curve of a recombinant protein as shown in SEQ ID NO: 14 acting on an LU5162B organoid system.
FIG. 13 shows a dose-response curve of a recombinant protein as shown in SEQ ID NO: 14 acting on an LU11624B organoid system.
FIG. 14 shows a dose-response curve of a recombinant protein as shown in SEQ ID NO: 14 acting on an LU1235B organoid system.
FIG. 15 shows a dose-response curve of a recombinant protein as shown in SEQ ID NO: 14 acting on an LI6677B organoid system.
FIG. 16 shows a dose-response curve of a recombinant protein as shown in SEQ ID NO: 14 acting on an LI6669B organoid system.
FIG. 17 shows a dose-response curve of a recombinant protein as shown in SEQ ID NO: 14 acting on a BR9457B organoid system.
FIG. 18 shows a dose-response curve of a recombinant protein as shown in SEQ ID NO: 14 acting on a BR9466B organoid system.
FIG. 19 shows a dose-response curve of a recombinant protein as shown in SEQ ID NO: 14 acting on a GA6833B organoid system.
FIG. 20 shows a dose-response curve of a recombinant protein as shown in SEQ ID NO: 14 acting on a GA2434B organoid system.
FIG. 21 shows a dose-response curve of a recombinant protein as shown in SEQ ID NO: 14 acting on a PA20078B organoid system.
FIG. 22 shows a dose-response curve of a recombinant protein as shown in SEQ ID NO: 14 acting on a PA0787B organoid system.
FIG. 23 shows changes in survival of mice in various treatment groups and control group in an LI6669 orthotopic transplantation tumor model of human liver cancer.
FIG. 24 shows a killing effect of a recombinant protein of rLZ-8 mutant on a lung cancer cell line A549.
FIG. 25 shows the comparison of expression results of recombinant proteins of rLZ-8 or a mutant linked via different spacer peptides, in which, M: marker; 1: rLZ-8 mutant 4; 2: EAEA+ rLZ-8 mutant 4 (SEQ ID NO: 25); 3: EEAEAEAEPK+ rLZ-8 mutant 4 (SEQ ID NO: 31); 4: rLZ-8 mutant 5; 5: EAEA+ rLZ-8 mutant 5 (SEQ ID NO: 26); 6: EEAEAEAEPK+ rLZ-8 mutant 5 (SEQ ID NO: 32); 7: rLZ-8 mutant 6; 8: EAEA+ rLZ-8 mutant 6 (SEQ ID NO: 27); 9: EEAEAEAEPK+ rLZ-8 mutant 6 (SEQ ID NO: 33).

### Detailed Description of the Embodiments

Hereinafter the embodiments of the invention of the application are described by specific examples. Those skilled in the art can easily understand other advantages and effects of the invention as described in the present application from the disclosure in the description.

### Definitions of Terms

In the present application, the term "recombinant protein" generally refers to a protein produced using a recombination technique. The recombinant protein can comprise: (1) a semi-synthetic or synthetic polypeptide produced by combined expression of DNA molecules from different sources linked by a recombinant DNA technique; (2) a polypeptide of semi-synthetic or synthetic origin, which is not associated with a moiety with which the polypeptide is associated in the naturally occurring state; (3) a polypeptide of semi-synthetic or synthetic origin, which is linked to a polypeptide other than those to which the polypeptide is linked in a naturally occurring state; or (4) a polypeptide of semi-synthetic or synthetic origin which does not exist in a naturally occurring state. For example, the recombinant protein can refer to a polypeptide formed by linking the spacer peptide of the present application to the recombinant *Ganoderma lucidum* immunomodulatory protein mutant (rLZ-8 mutant).

In the present application, the term "spacer peptide" generally refers to any oligopeptide or polypeptide that functions to link any domain. In the present application, the spacer peptide is located at the N-terminus of the recombinant *Ganoderma lucidum* immunomodulatory protein (rLZ-8). For example, the spacer peptide can be located between a leader peptide and the recombinant *Ganoderma lucidum* immunomodulatory protein (rLZ-8). For example, the spacer peptide can be a part of a signal peptide. For example, the spacer peptide can facilitate the secretion of the protein linked thereto.

In the present application, the term *"Ganoderma lucidum* immunomodulatory protein", also known as LZ-8, LZ8, Ling Zhi-8 or *Ganoderma lucidum* protein 8, generally refers to an immunomodulatory protein derived from *Ganoderma lucidum.* LZ-8 also comprise allelic variants, splice variants, derivative variants, substitution variants, deletion variants and/or insertion variants (including the addition of methionine at the N-terminus), fusion polypeptides and interspecific homologues thereof. In the present application, the LZ-8 can refer to an immunomodulatory protein derived *Ganoderma lucidum.* In the present application, the term "the recombinant *Ganoderma lucidum* immunomodulatory protein (rLZ-8)" generally refers to an LZ-8 obtained by a recombinant method. For example, the amino acid sequences of the LZ-8 and rLZ-8 can be found in UniProtKB Accession Number P14945. In the present application, the LZ-8 and rLZ-8 can comprise an amino acid sequence as shown in SEQ ID NO: 10.

In the present application, the term "mutant" generally refers to a sequence which is different from the reference sequence due to the inclusion of one or more differences. The reference sequence can be the amino acid sequence of the recombinant *Ganoderma lucidum* immunomodulatory protein. The differences can be deletion, insertion or preferably substitution of amino acid(s). The mutant can have the same or different functions with the reference sequence.

In the present application, the term "amino acid mutation" generally encompasses the substitution, deletion, insertion, and modification of one or more amino acids.

In the present application, the term "EA" generally refers to a peptide fragment formed by linking a glutamic acid (E) to an alanine (A) via peptide linkage.

In the present application, the term "isolated" generally refers to a biomaterial (e.g., virus, nucleic acid, or protein) substantially free of any component that usually accompany or interact therewith in a naturally occurring environment.

In the present application, the term "isolated nucleic acid molecule" generally refers to a genome, an mRNA, a cDNA, or a DNA or RNA of synthetic origin, or a combination thereof, which is not associated with all or a part of polynucleotides found in nature, or linked to a polynucleotide to which it is not linked in nature.

In the present application, the term "vector" generally refers to a nucleic acid molecule capable of self-replication in an appropriate host cell, which transfers an inserted nucleic acid molecule into a host cell and/or between host cells. The vector can comprise a vector mainly used for inserting a DNA or RNA into a cell, a vector mainly used for replicating a DNA or RNA, as well as a vector mainly used for the expression of transcription and/or translation of a DNA or RNA. The vector further comprise a vector having a plurality of functions as listed above. The vector can be a polynucleotide that can be transcribed and translated to a polypeptide when introduced into an appropriate host cell. In general, by culturing an appropriate host cell comprising the vector, the vector can produce a desired expression product.

In the present application, the term "cell" generally refers to an individual cell, cell line or cell culture that can comprise or have comprised a plasmid or vector comprising the nucleic acid molecule of the present application, or can express the antibody or its antigen-binding fragment of the present application. The cell can comprise a progeny of an individual cell. Due to a natural, accidental, or intentional mutation, the progeny cells are not necessarily the same as the original parent cells in morphology or genome, as along as they can express the antibody or an antigen binding fragment thereof of the present application. The cell can be obtained by *in vitro* transfecting a cell with the vector of the present application. The cell can be a prokaryotic cell (e.g., *Escherichia coli*), and can also be a eukaryotic cell (e.g., yeast cell, such as, a COS cell, a Chinese hamster ovary (CHO) cell, a HeLa cell, an HEK293 cell, a COS-1 cell, an NS0 cell, or a myeloma cell). In certain cases, the cell can be a mammalian cell. For example, the mammalian cell can be a CHO-K1 cell. In the present application, the term "recombinant cell" generally refers to a cell to which a recombinant expression vector is introduced. The recombinant host cell comprises not only a specific cell, but also the progeny of the cell.

In the present application, the term "kit" generally refers to a packaged product comprising components for administering the recombinant protein of the present application to treat disorders with EGFR expression and/or EGFR abnormal expression. The components of the kit are included in divided vials (that is, a kit having divided portions), or provided in a single vial. The kit can comprise an agent such as, buffer, protein-stabilized agent, signal-producing system (e.g., fluorescent signal-producing system), an antibody, a control protein, and a test vessel. The kit can further comprise an instruction for performing the method.

In the present application, the term "drug delivery device" comprises: (i) an infusion module for administering to a subject a pharmaceutical composition comprising an active ingredient; (ii) a pharmaceutical composition for infusion comprising an active ingredient selected from the group consisting of: a recombinant protein, a nucleic acid molecule, a cell, a vector or a combination thereof; and (iii) optionally a module for monitoring the drug efficiency.

In the present application, the term "tumor" generally refers to all the neoplastic cell growth and proliferation (whether malignant or benign) and all the precancerous and cancerous cells and tissues. The tumor can comprise a solid tumor and/or a non-solid tumor (e.g., hematologic tumor or lymphoma).

In the present application, the term "pharmaceutically acceptable carrier" generally comprises a pharmaceutically acceptable vector, excipient, or stabilizer, which is nontoxic to a cell or mammal exposed thereto at the utilized dose and concentration. In general, the physiologically acceptable vector is an aqueous pH buffer solution. Examples of the physiologically acceptable vector can comprise buffers, anti-oxidants, lower molecular weight (less than about 10 residues) polypeptides, proteins, hydrophilic polymers, amino acids, monosaccharides, disaccharides and other carbohydrates, chelating agents, sugar alcohols, salt-forming counterions such as sodium; and/or non-ionic surfactants.

In the present application, the term "treat/treatment/treating" generally refers to a clinical intervention which is expected to change the natural course of an individual to be treated, and can be used for prevention or can be carried out during the clinical pathological process. Desirable therapeutic effects comprise, but are not limited to, preventing the occurrence or reoccurrence of a disease, alleviating symptoms, weakening any direct or indirect pathological consequence of a disease, preventing metastasis, reducing the progression rate of a disease, improving or relieving the disease states, and remitting or improving the prognosis. In some cases, the recombinant vector of the present application can be used to delay the disease development or alleviate the disease progression.

In the present application, the term "administer/administering/administration" generally refers to a method of giving a certain dose of compound (e.g., an anti-cancer therapeutic agent) or pharmaceutical composition (e.g., a pharmaceutical composition comprising an anti-cancer therapeutic agent) to a subject (e.g., a patient). The administration can be carried out by any appropriate means, including parenteral, intrapulmonary, intranasal, and (if required in local treatment) intralesional administration. The parenteral infusion comprises, e.g., intramuscular, intravenous, intra-arterial, intra-peritoneal or subcutaneous administration.

In addition to the particular proteins and nucleotides as mentioned herein, the present application can further comprise functional variants, derivatives, analogs, homologues, or fragments thereof.

The term "functional variant" refers to a polypeptide which has an amino acid sequence that is substantially homologous to the naturally occurring sequence or is encoded by a nucleotide sequence that is substantially homologous to the naturally occurring sequence, and has one or more activities of the naturally occurring sequences. In the context of the present application, a variant of any give sequence refers to a sequence in which the particular sequence of residues (whether amino acid residues or nucleotide residues) has been modified so that the polypeptide or the polynucleotide substantially maintained at least one endogenous function. The variant sequence can be obtained by the addition, deletion, substitution, modification, replacement, and/or variation of at least one amino acid residue and/or nucleotide residue present in a naturally occurring protein and/or polynucleotide, as long as the original functional activity can be maintained.

In the present application, the term "derivative" generally refers to any substitution, variance, modification, replacement, deletion and/or addition of one (or more) amino acid residue(s) or nucleotide residue(s) in the polypeptide or polynucleotide of the present application, as long as the resultant polypeptide or polynucleotide substantially maintains at least one endogenous function thereof. In the present application, for a polypeptide or a polynucleotide, the term "analog" generally comprises any mimetic of the polypeptide or polynucleotide, that is, a chemical compound having at least one endogenous function of the polypeptide or polynucleotide mimicked by the mimetic. In general, amino acid substitution, e.g., at least 1 (e.g., 1, 2, 3, 4, 5, 6, 7, 8, 9, 10, 20 or greater) amino acid substitution, can be performed, as long as the modified sequence substantially maintains the desired activity or capacity. The amino acid substitution can comprise the use of a mimic that is not naturally occurring.

The protein and polypeptide for use in the present application can also have the deletion, insertion or substitution of amino acid residue which produces a silent change and results in a functionally equivalent protein. An intentional amino acid substitution can be performed in accordance with the similarity of polarity, charge, solubility, hydrophobicity, hydrophilicity and/or amphipathicity of the residues, as long as the endogenous function(s) can be maintained. For example, the negatively charged amino acids comprise aspartic acid and glutamic acid; the positively charged amino acids comprise lysine and arginine, and the amino acids which have similar hydrophilic values but do not have any electropolar head groups comprise asparagine, glutamine, serine, threonine, and tyrosine.

In the present application, the term "homologue" generally refers to an amino acid sequence or nucleotide sequence having a certain homology with a wild-type amino acid sequence or a wild-type nucleotide sequence. The term "homology" can equal to the sequence "identity". The homogeneous sequence can comprise an amino acid sequence having at least 80%, 85%, 90%, 99.1%, 99.2%, 99.3%, 99.4%, 99.5%, 99.6%, 99.7%, 99.8% or 99.9% to the subject sequence. In general, the homologues will comprise the same active sites as the subject amino acid sequence. The homology can be considered in accordance with the similarity (that is, amino acid residues with similar chemical properties/functions), or the homology can be expressed in terms of sequence homology. In the present application, a mentioned sequence with a percent homology with any one of the amino acid sequences or nucleotide sequences in SEQ ID NOs refers to a sequence with the percent homology in a full length of the SEQ ID NO as mentioned.

A sequence alignment can be carried out to determine the sequence homology, which can be performed by various methods known to persons skilled in the art, e.g., using BLAST, BLAST-2, ALIGN, NEEDLE or Megalign (DNASTAR) software, and the like. Those skilled in the art can determine suitable parameters for alignment, including any algorithms required to achieve the maximal alignment in the full length of the sequences to be compared.

In the present application, the term "and/or" can be understood to mean either or both of the options.

In the present application, the term "comprise/comprising/including" generally refers to including explicitly specified features, but not excluding other elements.

In the present application, the term "about" generally refers to a variation within 0.5%-10% of the specified value, e.g., a variation within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the specified value.

### DETAILED DESCRIPTION OF THE INVENTION

### Recombinant Protein

In an aspect, the present application provides a recombinant protein comprising a spacer peptide and a recombinant *Ganoderma lucidum* immunomodulatory protein(rLZ-8) mutant, wherein the rLZ-8 can comprise an amino acid sequence as shown in SEQ ID NO: 10. In the present application, the rLZ-8 can comprise an amino acid sequence having at least 80% (e.g., at least 85%, at least 90%, at least 95%, at least 96%, at least 97%, at least 98%, at least 99% or greater) homology with the amino acid sequence as shown in SEQ ID NO: 10.

In an aspect, the present application relates to a recombinant protein comprising a spacer peptide and a recombinant *Ganoderma lucidum* immunomodulatory protein mutant (rLZ-8 mutant), wherein compared with the amino acid sequence as shown in SEQ ID NO: 10, the amino acid sequence of the rLZ-8 mutant can comprise at least one amino acid mutation.

In the present application, the recombinant protein comprises a spacer peptide and a recombinant *Ganoderma lucidum* immunomodulatory protein mutant (rLZ-8 mutant), wherein compared with the amino acid sequence as shown in SEQ ID NO: 10, the amino acid sequence of the rLZ-8 mutant can comprise one amino acid mutation.

In the present application, the recombinant protein comprises a spacer peptide and a recombinant *Ganoderma lucidum* immunomodulatory protein mutant (rLZ-8 mutant), wherein compared with the amino acid sequence as shown in SEQ ID NO: 10, the amino acid sequence of the rLZ-8 mutant can comprise two amino acid mutations.

In the present application, the recombinant protein comprises a spacer peptide and a recombinant *Ganoderma lucidum* immunomodulatory protein mutant (rLZ-8 mutant), wherein compared with the amino acid sequence as shown in SEQ ID NO: 10, the amino acid sequence of the rLZ-8 mutant can comprise one amino acid mutation located at an amino acid position selected from the group consisting of: R9, L17, D20, D70, K46 and K74. For example, the one amino acid mutation can be located at an amino acid position selected from the group consisting of: D70, L17, K74 and K46.

In the present application, the amino acid mutation at L17 can be L17K.

In the present application, the amino acid mutation at D70 can be D70K.

In the present application, the amino acid mutation at K46 can be K46E.

In the present application, the amino acid mutation at K74 can be K74E.

In the present application, the amino acid mutation at D20 can be D20H.

In the present application, the amino acid mutation at R9 can be R9A.

In the present application, the recombinant protein comprises a spacer peptide and a recombinant *Ganoderma lucidum* immunomodulatory protein mutant (rLZ-8 mutant), wherein compared with the amino acid sequence as shown in SEQ ID NO: 10, the amino acid sequence of the rLZ-8 mutant can comprise one amino acid mutation selected from the group consisting of: R9A, L17K, D20H, K46E, D70K and K74E. Alternatively, e.g., the one amino acid mutation can be selected from the group consisting of: L17K, K46E, D70K and K74E.

In the present application, the recombinant protein comprises a spacer peptide and a recombinant *Ganoderma lucidum* immunomodulatory protein mutant (rLZ-8 mutant), wherein compared with the amino acid sequence as shown in SEQ ID NO: 10, the amino acid sequence of the rLZ-8 mutant can comprise one amino acid mutation selected from the group consisting of: L17K, D70K and K46E.

In the present application, the recombinant protein comprises a spacer peptide and a recombinant *Ganoderma lucidum* immunomodulatory protein mutant (rLZ-8 mutant), wherein compared with the amino acid sequence as shown in SEQ ID NO: 10, the amino acid sequence of the rLZ-8 mutant can comprise one amino acid mutation selected from the group consisting of: K46E and K74E. Alternatively, e.g., the two amino acid mutations can be selected from the group consisting of: L17K and D70K.

For example, the rLZ-8 mutant can comprise an amino acid sequence as shown in any one of SEQ ID NOS: 13 and 16-21. For example, the rLZ-8 mutant can comprise an amino acid sequence having at least 80% (e.g., at least 85%, at least 90%, at least 95%, at least 96%, at least 97%, at least 98%, at least 99% or greater) homology with the amino acid sequence as shown in any one of SEQ ID NOS: 13 and 16-21.

In the present application, the rLZ-8 mutant can comprise an amino acid sequence as shown in SEQ ID NO: 13. For example, the rLZ-8 mutant can comprise an amino acid sequence having at least 80% (e.g., at least 85%, at least 90%, at least 95%, at least 96%, at least 97%, at least 98%, at least 99% or greater) homology with the amino acid sequence as shown in SEQ ID NO 13. In the present application, the amino acid sequence of the rLZ-8 mutant can be as shown in SEQ ID NO: 13.

In the present application, the recombinant protein can comprise a spacer peptide and rLZ-8, wherein the rLZ-8 can comprise an amino acid sequence as shown in SEQ ID NO: 10, and the spacer peptide can comprise 1-5 EAs. For example, the spacer peptide can comprise 2 EAs.

In the present application, the recombinant protein can comprise a spacer peptide and an rLZ-8 mutant, wherein the rLZ-8 mutant can comprise an amino acid sequence as shown in any one of SEQ ID NOS: 13 and 16-21, the spacer peptide can comprise 1-5 EAs. For example, the spacer peptide can comprise 2 EAs.

In the present application, the recombinant protein can comprise a spacer peptide and an rLZ-8 mutant, wherein the rLZ-8 mutant can comprise an amino acid sequence as shown in SEQ ID NO: 13, and the spacer peptide can comprise an amino acid sequence as shown in SEQ ID NO: 8. For example, the recombinant protein can comprise an amino acid sequence as shown in SEQ ID NO: 14.

In the present application, the recombinant protein can comprise a spacer peptide and an rLZ-8 mutant, wherein the rLZ-8 mutant can comprise an amino acid sequence as shown in SEQ ID NO: 16, and the spacer peptide can comprise an amino acid sequence as shown in SEQ ID NO: 8. For example, the recombinant protein can comprise an amino acid sequence as shown in SEQ ID NO: 22.

In the present application, the recombinant protein can comprise a spacer peptide and an rLZ-8 mutant, wherein the rLZ-8 mutant can comprise an amino acid sequence as shown in SEQ ID NO: 17, and the spacer peptide can comprise an amino acid sequence as shown in SEQ ID NO: 8. For example, the recombinant protein can comprise an amino acid sequence as shown in SEQ ID NO: 23.

In the present application, the recombinant protein can comprise a spacer peptide and an rLZ-8 mutant, wherein the rLZ-8 mutant can comprise an amino acid sequence as shown in SEQ ID NO: 18, and the spacer peptide can comprise an amino acid sequence as shown in SEQ ID NO: 8. For example, the recombinant protein can comprise an amino acid sequence as shown in SEQ ID NO: 24.

In the present application, the recombinant protein can comprise a spacer peptide and an rLZ-8 mutant, wherein the rLZ-8 mutant can comprise an amino acid sequence as shown in SEQ ID NO: 19, and the spacer peptide can comprise an amino acid sequence as shown in SEQ ID NO: 8. For example, the recombinant protein can comprise an amino acid sequence as shown in SEQ ID NO: 25.

In the present application, the recombinant protein can comprise a spacer peptide and an rLZ-8 mutant, wherein the rLZ-8 mutant can comprise an amino acid sequence as shown in SEQ ID NO: 20, and the spacer peptide can comprise an amino acid sequence as shown in SEQ ID NO: 8. For example, the recombinant protein can comprise an amino acid sequence as shown in SEQ ID NO: 26.

In the present application, the recombinant protein can comprise a spacer peptide and an rLZ-8 mutant, wherein the rLZ-8 mutant can comprise an amino acid sequence as shown in SEQ ID NO: 21, and the spacer peptide can comprise an amino acid sequence as shown in SEQ ID NO: 8. For example, the recombinant protein can comprise an amino acid sequence as shown in SEQ ID NO: 27.

In the present application, the recombinant protein can comprise a spacer peptide and an rLZ-8 mutant, wherein the rLZ-8 mutant can comprise an amino acid sequence as shown in SEQ ID NO: 13, and the spacer peptide can comprise an amino acid sequence as shown in SEQ ID NO: 9. For example, the recombinant protein can comprise an amino acid sequence as shown in SEQ ID NO: 15.

In the present application, the recombinant protein can comprise a spacer peptide and an rLZ-8 mutant, wherein the rLZ-8 mutant can comprise an amino acid sequence as shown in SEQ ID NO: 16, and the spacer peptide can comprise an amino acid sequence as shown in SEQ ID NO: 9. For example, the recombinant protein can comprise an amino acid sequence as shown in SEQ ID NO: 28.

In the present application, the recombinant protein can comprise a spacer peptide and an rLZ-8 mutant, wherein the rLZ-8 mutant can comprise an amino acid sequence as shown in SEQ ID NO: 17, and the spacer peptide can comprise an amino acid sequence as shown in SEQ ID NO: 9. For example, the recombinant protein can comprise an amino acid sequence as shown in SEQ ID NO: 29.

In the present application, the recombinant protein can comprise a spacer peptide and an rLZ-8 mutant, wherein the rLZ-8 mutant can comprise an amino acid sequence as shown in SEQ ID NO: 18, and the spacer peptide can comprise an amino acid sequence as shown in SEQ ID NO: 9. For example, the recombinant protein can comprise an amino acid sequence as shown in SEQ ID NO: 30.

In the present application, the recombinant protein can comprise a spacer peptide and an rLZ-8 mutant, wherein the rLZ-8 mutant can comprise an amino acid sequence as shown in SEQ ID NO: 19, and the spacer peptide can comprise an amino acid sequence as shown in SEQ ID NO: 9. For example, the recombinant protein can comprise an amino acid sequence as shown in SEQ ID NO: 31.

In the present application, the recombinant protein can comprise a spacer peptide and an rLZ-8 mutant, wherein the rLZ-8 mutant can comprise an amino acid sequence as shown in SEQ ID NO: 20, and the spacer peptide can comprise an amino acid sequence as shown in SEQ ID NO: 9. For example, the recombinant protein can comprise an amino acid sequence as shown in SEQ ID NO: 32.

In the present application, the recombinant protein can comprise a spacer peptide and an rLZ-8 mutant, wherein the rLZ-8 mutant can comprise an amino acid sequence as shown in SEQ ID NO: 21, and the spacer peptide can comprise an amino acid sequence as shown in SEQ ID NO: 9. For example, the recombinant protein can comprise an amino acid sequence as shown in SEQ ID NO: 33.

In the present application, the spacer peptide can be located at an N-terminus of the rLZ-8 or a mutant thereof. In the present application, the recombinant protein can comprise in turn the spacer peptide and the rLZ-8 mutant from the N terminus to the C terminus.

In the present application, the recombinant protein can comprise a spacer peptide and rLZ-8, wherein the rLZ-8 mutant can comprise an amino acid sequence as shown in SEQ ID NO: 10, and the spacer peptide can comprise an amino acid sequence as shown in SEQ ID NO: 8.

In the present application, the recombinant protein can comprise a spacer peptide and rLZ-8, wherein the rLZ-8 mutant can comprise an amino acid sequence as shown in SEQ ID NO: 10, and the spacer peptide can comprise an amino acid sequence as shown in SEQ ID NO: 9.

In the present application, the spacer peptide can be located at an N-terminus of the rLZ-8. In the present application, the recombinant protein can comprise the spacer peptide and the rLZ-8 in turn from the N terminus to the C terminus.

In the present application, the recombinant protein can comprise an amino acid sequence as shown in any one of SEQ ID NOS: 14, 15 and 22-33. In the present application, the recombinant protein can comprise an amino acid sequence having at least 80% (e.g., at least 85%, at least 90%, at least 95%, at least 96%, at least 97%, at least 98%, at least 99% or greater) homology with the amino acid sequence as shown in any one of SEQ ID NOS: 14, 15, and 22-33.

For example, the recombinant protein can comprise an amino acid sequence as shown in SEQ ID NO: 14. For example, the recombinant protein can comprise an amino acid sequence having at least 80% (e.g., at least 85%, at least 90%, at least 95%, at least 96%, at least 97%, at least 98%, at least 99% or greater) homology with the amino acid sequence as shown in SEQ ID NO: 14.

### Nucleic Acid, Vector, and Cell

In another aspect, the present application provides a polypeptide comprising the recombinant protein. In the polypeptide, the recombinant protein can be covalently or non-covalently linked to other proteins or polypeptides.

In another aspect, the present application provides one or more nucleic acid molecules that can encode the recombinant protein and/or polypeptide of the present application. The nucleic acid molecule of the present application can be isolated. For example, it can be produced or synthesized: (i) by *in vitro* amplification, such as, by polymerase chain reaction (PCR) amplification, (ii) by clonal recombination, (iii) by purification, e.g., by enzyme digestion and gel electrophoresis fractionation, or (iv) by synthesis, e.g., by chemical synthesis. In some embodiments, the isolated nucleic acid is a nucleic acid molecule prepared by recombinant DNA technology.

In another aspect, the present application provides a vector that can comprise the nucleic acid molecule of the present application. Moreover, the vector can further comprise other genes, such as, a marker gene that allows the vector to be selected in a suitable host cell under suitable conditions. Moreover, the vector can further comprise an expression control element that allows the coding region to be properly expressed in a suitable host. Such control elements can be well known by persons skilled in the art, e.g., they can comprise promoters, ribosome binding sites, enhancers, and other control elements regulating the transcription of genes or the translation of mRNAs, etc. The vector can comprise, e.g., plasmids, cosmids, viruses, phages, or other vectors commonly used in genetic engineering, etc. For example, the vector is an expression vector. For example, the vector is a vector suitable for the expression of eukaryocyte (e.g., yeast cell).

In another aspect, the present application provides a cell that can comprise the nucleic acid molecule of the present application or the vector of the present application. In certain embodiments, each cell can comprise one nucleic acid molecule or vector of the present application. In certain embodiments, each cell can comprise more than one (e.g., 2 or more) nucleic acid molecules or vectors of the present application. For example, the vector of the present application can be introduced into the host cell, such as, a yeast cell, e.g., a cell from plant, a fungal cell or a yeast cells, etc. The vector of the present application can be introduced into the host cell by a method known in the art, e.g., electroporation, lipofectine transfection, lipofectamin transfection, etc.

In another aspect, the present application provides a method of preparing the recombinant protein comprising culturing the cell under conditions that allow to express the recombinant protein. For example, it can be done by using an appropriate medium, an appropriate temperature and culturing time, etc.

### Pharmaceutical Composition, Kit and Drug Delivery Device

In another aspect, the present application provides a pharmaceutical composition comprising the recombinant protein, the isolated nucleic acid molecule, the vector, the polypeptide and/or the cell, and optionally a pharmaceutically acceptable carrier. The pharmaceutically acceptable vector is nontoxic to the receipt at the used dose and concentration, and can comprise buffers, anti-oxidants, preservatives, lower molecular weight (less than about 10 residues) polypeptides, proteins, hydrophilic polymers, amino acids, carbohydrates, salt-forming counterions, metal complexes, and/or non-ionic surfactants. The pharmaceutical composition of the present application can further comprise more than one active compound, generally, those active compounds that have complementary activities and would not would not adversely affect each other. The type and effective amount of such drugs depend on, e.g., the amount and type of antagonist(s) present in the formulation, and the clinical parameters of the subject. The pharmaceutical composition of the present application can comprise a prophylactically and/or therapeutically effective amount of the recombinant protein and/or polypeptide. The prophylactically and/or therapeutically effective amount is a dose required to prevent and/or treat (at least partially treat) a disease or disorder and or any complication thereof in a subject suffering from or at a risk of development.

In another aspect, the present application provides a kit comprising the recombinant protein, the isolated nucleic acid molecule, the vector, the cell, the polypeptide and/or the pharmaceutical composition. It is feasible that the antigen-binding protein, vector, nucleic acid molecule, cell, immunoconjugate and/or the pharmaceutical composition of the present application can be contained in a single common container, or they can also be optionally combined with one or more therapeutic agents and optionally formulated together in a kit.

In another aspect, the present application provides a drug delivery device comprising the recombinant protein, the isolated nucleic acid molecule, the vector, the cell, the polypeptide and/or the pharmaceutical composition.

### Method of Treatment

In another aspect, the present application provides a method of preventing, alleviating or treating a tumor comprising administering to a subject in need thereof the recombinant protein, the isolated nucleic acid molecule, the vector, the cell, the polypeptide and/or the pharmaceutical composition. In another aspect, the present application provides use of the recombinant protein, the isolated nucleic acid molecule, the vector, the cell, the polypeptide and/or the pharmaceutical composition in the manufacture of a drug for treating, alleviating or treating a tumor.

In another aspect, the present application provides the recombinant protein, the isolated nucleic acid molecule, the vector, the cell, the polypeptide and/or the pharmaceutical composition for preventing, alleviating or treating a tumor.

For example, the tumor can comprise a solid tumor and/or a non-solid tumor (e.g., hematologic tumor).

For example, the tumor can be a tumor with EGFR expression. For example, the tumor can be a tumor associated with abnormal EGFR expression. For example, the tumor can be a tumor with EGFR overexpression.

For example, the tumor can comprise a tumor associated with EGFR mutation. For example, the tumor can comprise a tumor associated with BRAF mutation. For example, the tumor can comprise a tumor associated with KRAS mutation. For example, the tumor can comprise a tumor associated with HER2 mutation.

For example, the tumor can comprise one or more selected from the group consisting of: colorectal cancer, lung cancer, liver cancer, breast cancer, gastric cancer, renal cancer, bladder cancer, neuroblastoma, ovarian cancer, epithelial squamous carcinoma, and pancreatic cancer.

Without wishing to be bound by any theory, the following examples are only for illustrating various technical solutions of the invention of the present application, and are not intended to limit the scope of the invention of the application.

### EXAMPLES

### Example 1: Construction of Expression Vector of rLZ-8 and rLZ-8 Mutant

(1) The rLZ-8 (with an amino acid sequence as shown in SEQ ID NO: 10) DNA and the rLZ-8 mutant (with an amino acid sequence as shown in SEQ ID NO: 13) DNA were taken as template, and the forward primer F and the reverse primer R were used for the PCR amplification of the gene fragments of the rLZ-8 and the rLZ-8 mutant. The obtained gene fragments did not comprise a DNA sequence of EAEA, but had Xho I and Xba I digestion sites at 5' end and 3' end, respectively.

The information of the forward primer F and the reverse primer R are as follows:
The forward primer F: CCGCTCGAGAAAAGAATGTCTGATACTGCTTTGATCTTCA (SEQ ID NO: 1)
The reverse primer R: GCTCTAGACTAGTTCCATTGAGCGATA (SEQ ID NO: 2)

(2)The gene fragments of the rLZ-8 (with an amino acid sequence as shown in SEQ ID NO: 10) and the rLZ-8 mutant (with an amino acid sequence as shown in SEQ ID NO: 13) were digested by Xho I/Xba I, and ligated into an empty expression vector by T4 DNA ligase to obtain a recombination expression vector.

### Example 2: Construction of Expression Vector of rLZ-8 and rLZ-8 Mutant With EAEA Sequence

(1) In accordance with the codon preference of *Pichia pastoris,* with overall consideration of influences such as codon adaptation index and RNA secondary structure analysis and the like, the EAEA+rLZ-8 (with an amino acid sequence as shown in SEQ ID NO: 11) and the EAEA+rLZ-8 mutant (with an amino acid sequence as shown in SEQ ID NO: 14) were subj ect to codon optimization. To facilitate the construction of expression vector, Xho I and Xba I restriction enzyme cutting sites were added to the 5' and 3' end of the optimized DNA sequence, respectively, and the DNA sequence was loaded to a pUC57 clonal vector after artificial synthesis, that is, forming the pUC57-EAEA+rLZ-8 and the pUC57- EAEA+rLZ-8 mutant.
(2) The EAEA+rLZ-8 (with an amino acid sequence as shown in SEQ ID NO: 11) DNA was taken as template, and the forward primer F3 and the reverse primer R3 were used for PCR amplification of the gene fragments of the rLZ-8.The obtained gene fragments comprised a DNA sequence with EAEA, and had Xho I and Xba I digestion sites at 5' end and 3' end, respectively.

The information of the forward primer F3 and the reverse primer R3 are as follows:

The forward primer F3: CCGCTCGAGAAAAGAGAGGCTGAAGCT (SEQ ID NO: 3)

The reverse primer R3: GCTCTAGATCACTAGTTCCATTG (SEQ ID NO: 4)

(3) The EAEA+rLZ-8 mutant (with an amino acid sequence as shown in SEQ ID NO: 14) DNA was taken as template, and the forward primer M13F and the reverse primer M13R were used for PCR amplification of the gene fragments of the rLZ-8 mutant. The obtained gene fragments comprised a DNA sequence with EAEA, and had Xho I and Xba I digestion sites at 5' end and 3' end, respectively.

The information of the forward primer M13F and the reverse primer M13R are as follows:

The forward primer M13F: CCCAGTCACGACGTTGTAAAACG (SEQ ID NO: 5)

The reverse primer M13R: AGCGGATAACAATTTCACACAGG (SEQ ID NO: 6)

(4) The gene fragments of the EAEA+rLZ-8 and the EAEA+rLZ-8 mutant were digested by Xho I/Xba I, and ligated into an empty expression vector by T4 DNA ligase to obtain a recombination expression vector.

Using the same method, recombinant proteins comprising the rLZ-8 mutant 1 (with an amino acid sequence as shown in SEQ ID NO: 16), the rLZ-8 mutant 2 (with an amino acid sequence as shown in SEQ ID NO: 17), the rLZ-8 mutant 3 (with an amino acid sequence as shown in SEQ ID NO: 18), the rLZ-8 mutant 4 (with an amino acid sequence as shown in SEQ ID NO: 19), the rLZ-8 mutant 5 (with an amino acid sequence as shown in SEQ ID NO: 20), the rLZ-8 mutant 6 (with an amino acid sequence as shown in SEQ ID NO: 21) linked to the amino acid sequence "EAEA" at the N-terminus were constructed, respectively.

### Example 3: Construction of Expression Vector of rLZ-8 and rLZ-8 Mutant With EEAEAEAEPK Sequence

(1) The EEAEAEAEPK+rLZ-8 DNA (with an amino acid sequence as shown in SEQ ID NO: 12) and the EEAEAEAEPK+rLZ-8 mutant (with an amino acid sequence as shown in SEQ ID NO: 15) DNA were taken as template, and the forward primer F2 and the reverse primer R were used for the PCR amplification of the gene fragments of the EEAEAEAEPK+rLZ-8 and the EEAEAEAEPK+rLZ-8 mutant. The obtained gene fragments comprised a DNA sequence of EEAEAEAEPK, and had Xho I and Xba I digestion sites at 5' end and 3' end, respectively.

The information the forward primer F2 and the reverse primer R are as follows:
The forward primer F2:

CCGCTCGAGAAAAGAGAAGAGGCCGAAGCCGAAGCAGAACCTAAAATGTCTGATA
CTGCTTTGATCTTCA (SEQ ID NO: 7)

The reverse primer R: GCTCTAGACTAGTTCCATTGAGCGATA (SEQ ID NO: 2)
(2)The gene fragments of the EEAEAEAEPK+rLZ-8 (with an amino acid sequence as shown in SEQ ID NO: 12) and the EEAEAEAEPK+rLZ-8 mutant (with an amino acid sequence as shown in SEQ ID NO: 15) were digested by Xho I/Xba I, and ligated into an empty expression vector by T4 DNA ligase to obtain a recombination expression vector.

Using the same method, recombinant proteins comprising the rLZ-8 mutant 1 (with an amino acid sequence as shown in SEQ ID NO: 16), the rLZ-8 mutant 2 (with an amino acid sequence as shown in SEQ ID NO: 17), the rLZ-8 mutant 3 (with an amino acid sequence as shown in SEQ ID NO: 18), the rLZ-8 mutant 4 (with an amino acid sequence as shown in SEQ ID NO: 19), the rLZ-8 mutant 5 (with an amino acid sequence as shown in SEQ ID NO: 20), the rLZ-8 mutant 6 (with an amino acid sequence as shown in SEQ ID NO: 21) linked to the amino acid sequence "EEAEAEAEPK" at the N-terminus were constructed, respectively.

### Example 4: Construction of Pichiapastoris Expression Strain

### (1) Preparation of Competent Cell:

Empty strains of *Pichia pastoris* were inoculated into a YPD fluid medium, and cultured at 28.5 °C for 16-18h until OD₆₀₀=1.3-1.5 so that the competent cells were prepared. The strain solution were placed in an ice bath for 30 min, and sub-packed into four 50-mL centrifuge tubes, each of which contained 40 mL. Centrifuge was performed at 1500 g at 4 °C for 5 min. The supernatant was discarded. The cells were collected, fully re-suspended in 160 mL of pre-cooled sterile double distilled water, and centrifuged at 1500 g at 4 °C for 5 min. The supernatant was discarded again. The cells were collected, fully re-suspended in 80 mL of pre-cooled sterile double distilled water, and centrifuged at 1500 g at 4 °C for 5 min. The cells were collected, fully re-suspended in 10 mL of 1 M pre-cooled sterile sorbitol solution, and centrifuged at 1500 g at 4 °C for 5 min. The cell precipitate was collected and re-suspended in 500 µL of 1 M pre-cooled sterile sorbitol solution for later use.

### (2) Linearization of Recombinant Plasmid

The recombinant plasmid was linearized with Bsp H I to facilitate transformation into yeast cells.

### (3) Electrotransformation

80 µL of competent yeast cells were added into a 2 mm electrotransformation beaker. 5-10 µg of the linearized recombinant plasmids were added, mixed well, and stood on ice for 5 min. The electrotransformation parameters are set as follows: V=1.5 kv, u=25 µF, R=200 Ω, and the discharge time is between 4.5 ms and 4.9 ms. After electrotransformation, 1 mL of 1 M Sorbitol was immediately added, and incubated at a constant temperature of 28.5 °C for 1 h. 50-200 µL of the strain solution was applied onto a YPDS solid medium plate containing 100 µg/mL of Zeocin resistance. It was incubated in a constant temperature incubator at 28.5 °C for 2-3 days.

### Example 5: Screening of Strains with High Expression

Zeocin positive clonal colony was inoculated into 10 mL of YPD fluid medium for culture. The OD value was detected with a spectrophotometer at 600 nm until the OD value ≈ 6. 40 µl of activated strain solution was inoculated into 25 mL of fresh YPD fluid medium, and cultured at 28.5 °C and 225 rpm for 72 h. 1 mL of strain solution was centrifuged at 13000 g at low temperature of 4 °C, and the supernatant was detected by SDS-PAGE electrophoresis for the expression of multiple clones of the target protein in 6 expression vectors constructed in Examples 1-3.

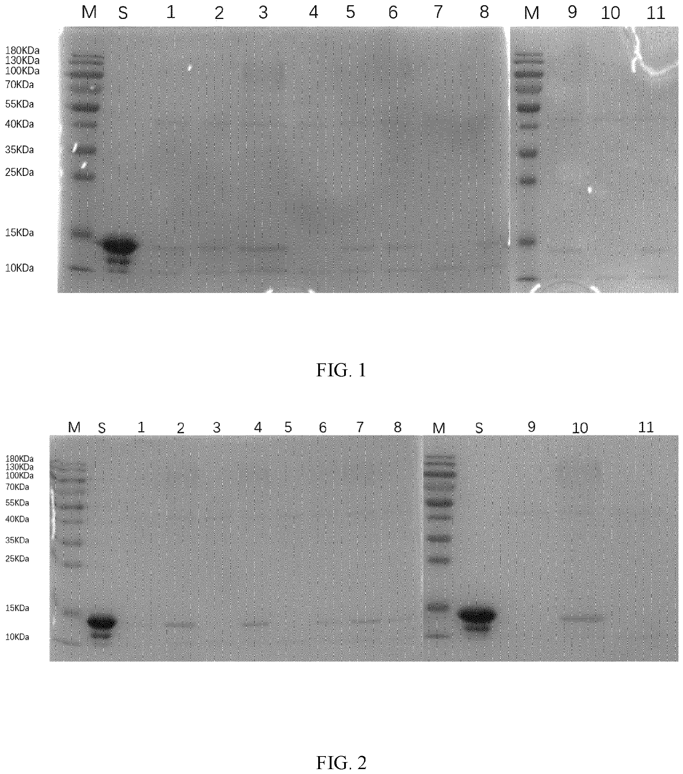

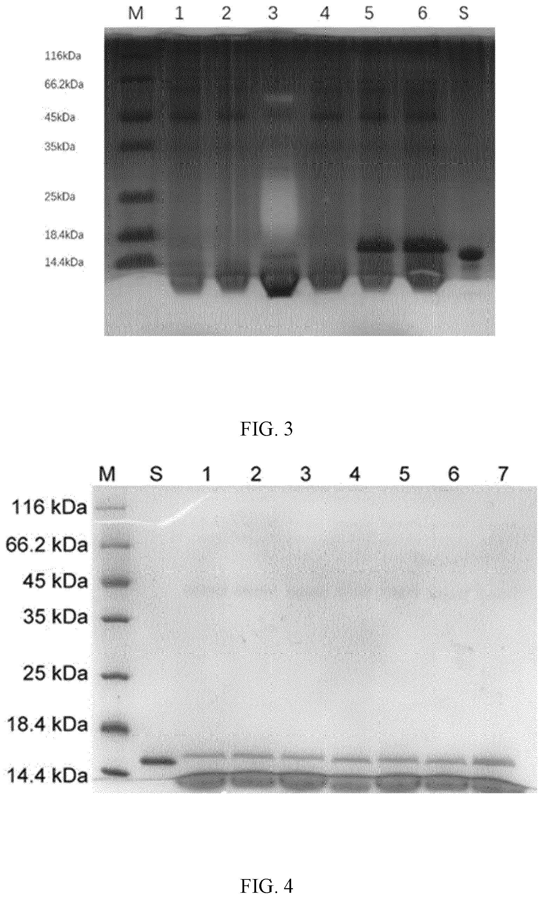

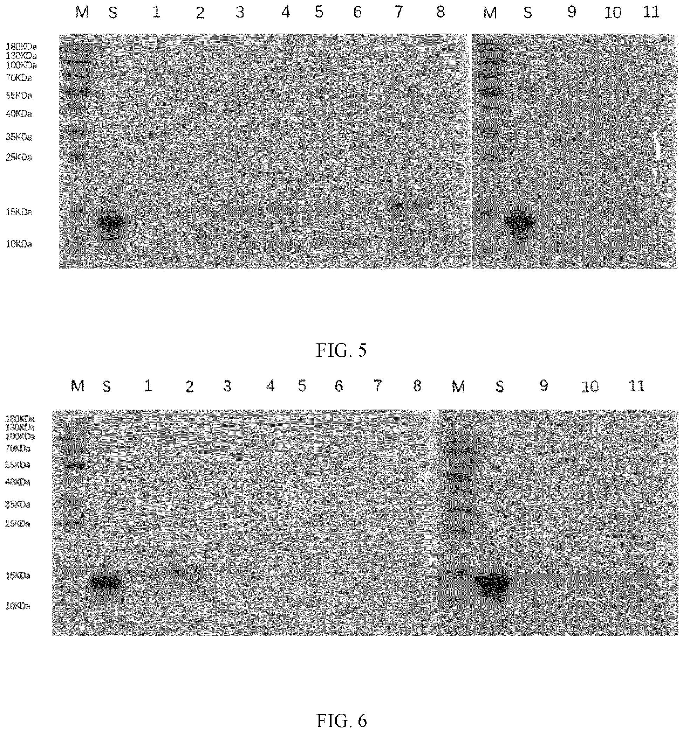

The screening results of the yeast strains with high expression are shown in Figs. 1-6. As for the rLZ-8 (SEQ ID NO: 10), Clone 3 has a relatively high expression (FIG. 1); as for the rLZ-8 mutant (SEQ ID NO: 13), Clone 10 has a relatively high expression (FIG. 2); as for the EAEA+rLZ-8 (SEQ ID NO: 11), Clone 6 has a relatively high expression (FIG. 3); as for the EAEA+rLZ-8 mutant (SEQ ID NO: 14), Clone 7 has a relatively high expression (FIG. 4); as for the EEAEAEAEPK+rLZ-8 (SEQ ID NO: 12), Clone 7 has a relatively high expression (FIG. 5); and as for the EEAEAEAEPK+rLZ-8 mutant (SEQ ID NO: 15), Clone 2 has a relatively high expression (FIG. 6).

### Example 6: Effect of Different Spacers on Protein Expression of rLZ-8 and rLZ-8 Mutant

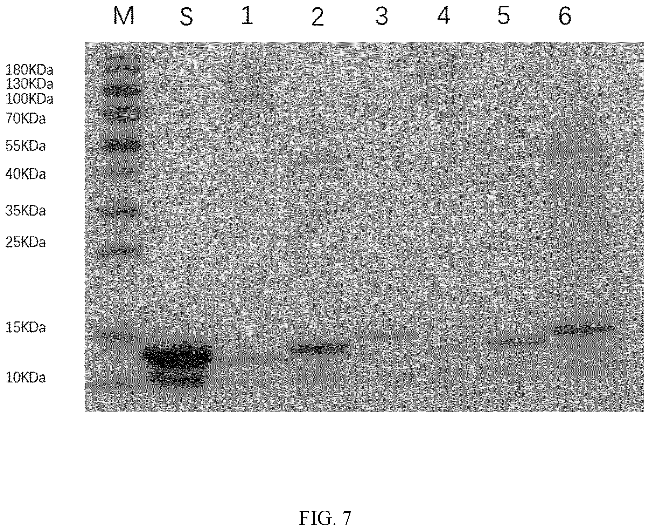

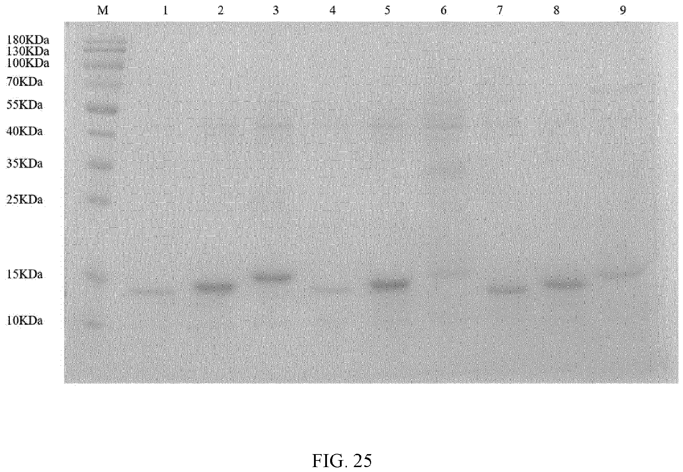

(1) The expressions of the target proteins in multiple expression vectors constructed in Examples 1-3 were compared in accordance with the method of Example 5. The results are shown in FIG. 7.
   EAEA has an effect promoting the expression and secretion of both the rLZ-8 (SEQ ID NO: 10) and the rLZ-8 mutant (SEQ ID NO: 13), wherein the promoting effect on the rLZ-8 mutant (SEQ ID NO: 13) is better than that on the rLZ-8 (SEQ ID NO: 10) (Lanes 1, 2, 4 and 5 in FIG. 7).
   EEAEAEAEPK has an effect of promoting the expression and secretion of both the rLZ-8 (SEQ ID NO: 10) and the rLZ-8 mutant (SEQ ID NO: 13), wherein the promoting effect on the rLZ-8 (SEQ ID NO: 10) is better than that on the rLZ-8 mutant (SEQ ID NO: 13) (Lanes 1, 3, 4 and 6 in FIG. 7).
   EAEA and EEAEAEAEPK both have an effect of increasing the expression and secretion of protein, and their promoting effects on the expression and secretion on different proteins are different, wherein EAEA has an optimal promoting effect on the rLZ-8 mutant (SEQ ID NO: 13) (Lanes 1, 2 and 3 in FIG. 7), but EEAEAEAEPK has an optimal promoting effect on the rLZ-8 (SEQ ID NO: 10) (Lanes 4, 5 and 6 in FIG. 7).
(2) The expressions of the target proteins (based on the rLZ-8 mutant 4 to rLz-8 mutant 6) in 9 expression vectors constructed in Examples 1-3 were compared in accordance with the method of Example 5. The results are shown in FIG. 25.

The results show that the EAEA has an effect of increasing the expression and secretion of protein, and has optimal effects on the rLZ-8 mutant 4 (SEQ ID NO: 19), the rLZ-8 mutant 5 (SEQ ID NO: 20) and rLZ-8 mutant 6 (SEQ ID NO: 21).

### Example 7. Identification of Structure of Recombinant Protein

This example determined the N-terminal amino acid sequences of the EAEA+rLZ-8 (SEQ ID NO: 11), the EAEA+rLZ-8 mutant (SEQ ID NO: 14), the EEAEAEAEPK+ rLZ-8 (SEQ ID NO: 12), the EEAEAEAEPK+ rLZ-8 mutant (SEQ ID NO: 15), the rLZ-8 (SEQ ID NO: 10) and the rLZ-8 mutant (SEQ ID NO: 13) proteins. The recombinant protein was hydrolyzed by trypsin, and then isolated by reverse chromatography to obtain peptide segments produced by hydrolysis, each of which was measured by mass spectrometry to obtain the N-terminal amino acid sequence. The particular steps were as follows:
(1) Preparation of Sample Solution
   250 µg of recombinant protein was added into 200 µl of 10 M urea solution, and diluted with water to 250 µl. After mixed well, it was incubated in a constant-temperature mixer at 65 °C for 30 min. After incubation, the sample was cooled to room. The buffer solution was replaced with 0.1 M ammonium bicarbonate solution by SEC column. The final protein concentration was measured by micro-spectrophotometer.
(2) Enzymolysis
   50 µg protein to be tested was diluted with 0.1M ammonium bicarbonate solution to 0.5 µg/µl. 0.5 µg of trypsin was added and mixed well, and then incubated at 37 °C for 20 h. After incubation, the enzymolysis reaction was stopped by adding an equivalent volume of 0.1% formic acid solution or standing at 4 °C. The solution was centrifuged at 4 °C at 12000 rpm for 2 min, and the supernatant was sampled for analysis.
(3) Chromatographic Conditions and Elution Procedures

### 3.1 Chromatographic Conditions

| | |
|---|---|
| Column: | ACQUITY UPLC CSH C18 Column 2.1* 150mm 1.7µm |
| Mobile Phase A: | 0.1% formic acid in water |
| Mobile Phase B: | 0.1% formic acid in acetonitrile |
| Elution Mode: | Gradient Elution |
| Flow rare: | 0.3 mL/min |
| Elution Time: | 75 min |
| Column temperature: | 55 °C |
| Detection Wavelength: | 214 nm |
| Loading: | 10 µl |

### 3.2 Elution Procedures

| Time (min) | Organic phase (%) |
|---|---|
| 0 | 2 |
| 3 | 2 |
| 63 | 40 |
| 63.1 | 90 |
| 66 | 90 |
| 66.1 | 2 |
| 75 | 2 |

The results show that the N-terminal amino acid sequences of the recombinant proteins are all consistent with the theoretical sequences.

### Example 8. Detection of Killing Effects of EAEA+rLZ-8 Mutant and EEAEAEAEPK+rLZ-8 Mutant on Tumor Cells

CellTiterTM-Flour Cell Viability Assay Reagent kit was used to detect the killing effects of the EAEA+ rLZ-8 mutant (SEQ ID NO: 14) and the EEAEAEAEPK+rLZ-8 mutant (SEQ ID NO: 15) on tumor cells (human non-small cell lung cancer cell line A549) *in vitro.* The recombinant protein was diluted with 2% fetal bovine serum culture medium to produce test solutions with concentrations of 100 µg/mL, 50 µg/mL, 25 µg/mL, 12.5 µg/mL, 6.25 µg/mL, 3.125 µg/mL, 1.5625 µg/mL, 0.78125 µg/mL, 0.390625 µg/mL, 0.1953125 µg/mL, and 0.09765625µg/mL, total 11 concentrations.

The dilutions were added into a 96-well plate containing A549 cells with 100 µL/well. For each group of concentration, 3 parallel wells were set and marked. A cell control group (i.e., cells only) was set at 100 µL/well, with 6 parallel wells. The plates were cultured in a CO₂ incubator at 5% CO₂ and 37 °C for 48 h.

100 µL of prepared CellTiterTM-Flour Cell Viability Assay Reagent (1 mLL Assay Buffer; 1 µl GF-AFC Substrate) was added into each well. The plates were shaken for mixing well, and incubated in a CO₂ incubator at 5% CO₂ and 37 °C for not less than 30 min. The fluorescence value was detected with a microplate reader at a wavelength of 400EX/505EM, and the IC₅₀value was calculated.

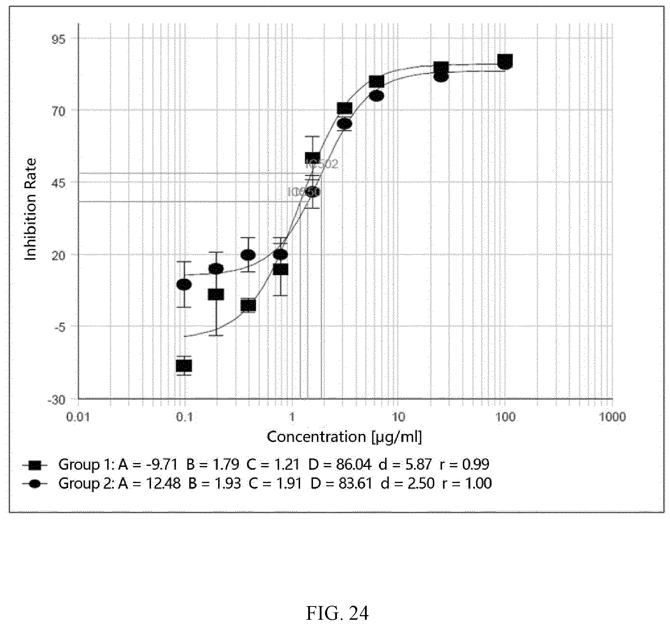

The results are shown in FIG. 24 (wherein Group 1 and Group 2 correspond to the results of the EAEA+rLZ-8 mutant (SEQ ID NO: 14) and the EEAEAEAEPK+rLZ-8 mutant (SEQ ID NO: 15), respectively). The IC₅₀ of the EAEA+rLZ-8 mutant (SEQ ID NO: 14) is 1.21 µg/ml; and the IC₅₀ of the EEAEAEAEPK+rLZ-8 mutant (SEQ ID NO: 15) is 1.91 µg/ml. The results show that both the EAEA+rLZ-8 mutant (SEQ ID NO: 14) and the EEAEAEAEPK+rLZ-8 mutant (SEQ ID NO: 15) have killing effect on the lung cancer cell line A549, wherein the killing effect of the EAEA+rLZ-8 mutant (SEQ ID NO: 14) is superior to that of the EEAEAEAEPK+rLZ-8 mutant (SEQ ID NO: 15).

### Example 9. In Vitro Inhibition of Recombinant Protein on Tumor Growth (PDXO Model)

On the day before test (Day -1), 50% Matrigel was used to treat the desired number of organoids at a ratio of 1:1 to define the proper dimension of the organoid for screening.

### Day 0: Organoid Inoculation

a) 20 µl of 100x Dispase solution was added in to a 6-well plate (containing 2 mL of organism culture medium), and organoid was collected from each well. b) The 6-well plate was returned into the incubator and cultured at 37 °C for 30 min. c) The organoid was collected from the 6-well plate, and transferred by a pre-wetted 100 µm filter into a 50 mL plastic tube. d) Once all the wells were filtered by 100 µm filter, the flow was filtered by a pre-wetting 20 µm filter, e) The 20 µm filter was inverted, and the organoid was collected in a new 50 mL tube. f) The organoid was collected and re-suspended in the corresponding culture medium. The organoid was calculated for their concentrations. g) The cell concentration was adjusted with the medium to an appropriate concentration, h) Matrigel was added to a final concentration of 5% v/v, and the organoid was suspended on ice. i) 40 µL of cell suspension was added with a Multidrop dispenser into a 384-well plate. The inoculation density is listed in Table 1 below, which is associated with the morphology and growth conditions of the organoid. j) After addition of the recombinant protein, the screening plates were returned into the incubator. 2-4 hours after inoculation, a solution of the recombinant protein was added to treat the organoid. The amino acid sequence of the spacer of the recombinant protein used in the example is shown in SEQ ID NO:8, the amino acid sequence of the rLZ-8 mutant is shown in SEQ ID NO: 13, and the amino acid sequence of the recombinant protein is shown in SEQ ID NO: 14.

**Table 1. Inoculation Density of Organoid**

| Organoid Line | Inoculation Density |
|---|---|
| LI6669B B | 500/well |
| GA6833B | 350/well |
| CR3099B | 400/well |
| CR20035B | 2000/well |
| GA2434B | 350/well |
| LU5162B | 350/well |
| LI6677B | 400/well |
| CR5043B | 400/well |
| CR5082B | 400/well |
| PA20078B | 400/well |
| PA0787B | 400/well |
| LU11624B | 400/well |
| LU1235B | 400/well |
| BR9457B | 3000/well |

Day 5: The luminous CTG signal of the detection plate was read at the end of detection: 40 µL of CTG 3D was added into each well by a Multidrop dispenser; the content was mixed on a plate oscillator for 5 min; and then the place was incubated at room temperature in dark for 30 min. The luminous signal was read on an Envision plate reader.

### Analysis of Data:

The data will be graphically displayed using GraphPad Prism 5.0. The formula for calculating the survival rate is as follows.

Survival Rate (%) = (Lum_{Test article} - Lum_{Medium control}) / (Lum_{None treated} - Lum_{Medium control}) × 100%. The medium control is the same as the positive control (Staurosporine, 5 µM), because the positive control (Staurosporine, 5 µM) is set to kill all or almost all organoids.

### (1) Colorectal Cancer

**Table 2. Inhibition of Recombinant Protein on Growth of Colorectal Cancer**

| Organ oid Line | EGFR Expressi on (IHC) | Amino Acid Mutatio n | IC50 µg/ml (the recombina nt protein as shown in SEQ ID NO: 14) | Maxim um Inhibiti on Rate (%) (the recomb inant protein as shown in SEQ ID NO:14) | IC50 µg/ml (Cetuxi mab) | Maxim um Inhibiti on Rate (%) (Cetuxi mab) | IC50 µM (Staurosp orine) | Maximu m Inhibition Rate (%) (Staurosp orine) |
|---|---|---|---|---|---|---|---|---|
| CR20 035B | 119.7401 66 | NA | 70.3983 | 99.99 | NA | NA | 0.0011 | 100.04% |
| CR50 43B | 170.4053 19 | KRAS G13D | 75.241 | 87.88 | 277.853 | 93.67 | 0.107 | 100.00 |
| CR50 82B | 265.8223 57 | BRAF V600E | 87.672 | 97.53 | 459.8 | 90.92 | 0.010 | 99.98 |
| CR30 99B | 25.32491 3 | EGFR (p.R52 1K) / KRAS (p.G12 D) | 68.374 | 92.37 | >500 | 47.57 | 0.0049 | 100.64 |

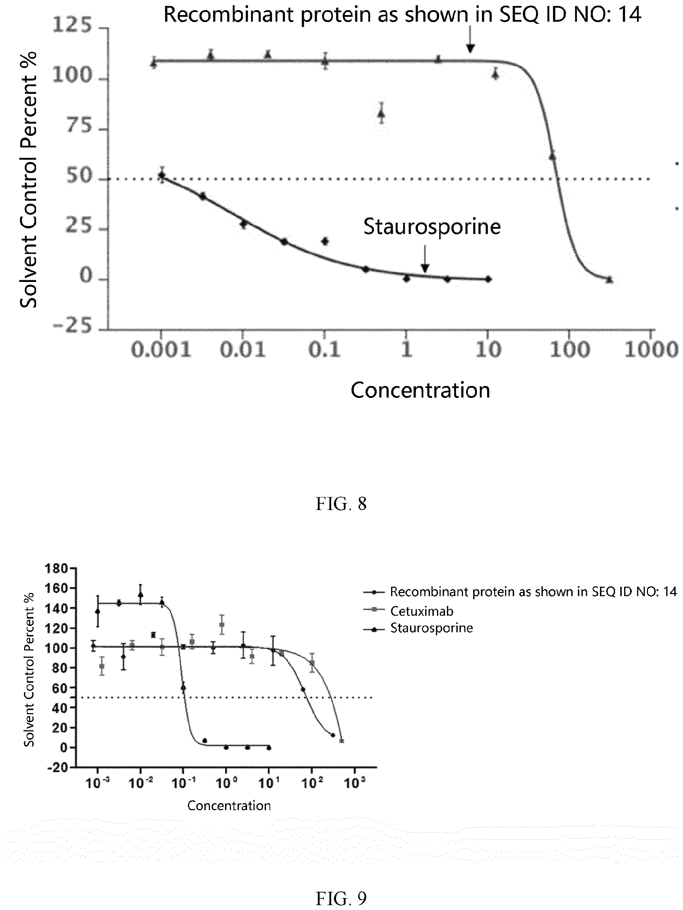

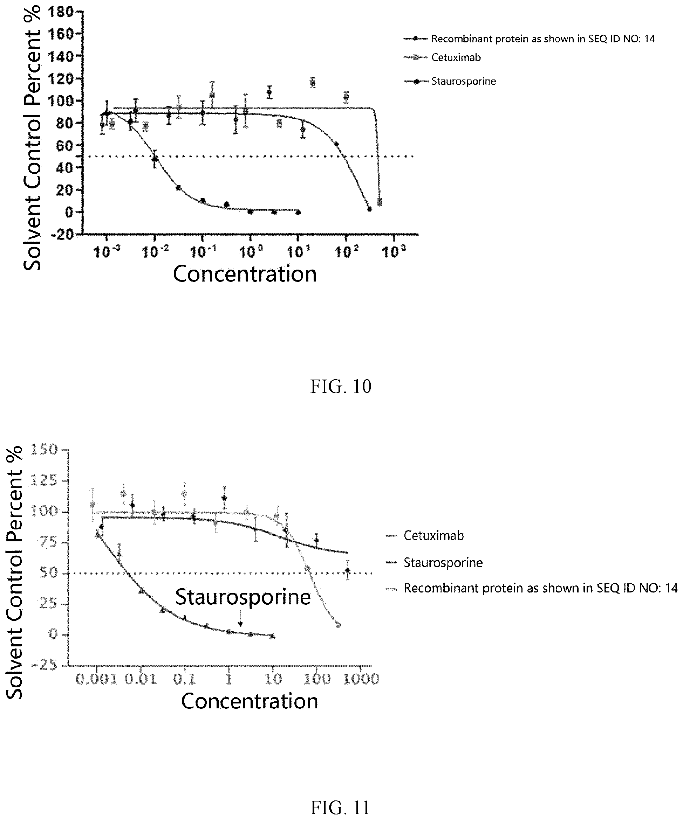

The results are shown in Table 2, and FIG. 8 to FIG. 11: The recombinant protein as shown in SEQ ID NO: 14 has inhibitory effects on the colorectal cancer cells with EGFR wild-type (CR20035B, FIG. 8), KRAS mutation (CR5043B, FIG. 9), BRAF mutation (CR5082B, FIG. 10), as well as the simultaneous mutation of EGFR and KRAS (CR3099B, FIG. 11).

### (2) Lung Cancer

**Table 3. Inhibition of Recombinant Protein on Growth of Lung Cancer**

| Organoid Line | EGFR Expression (IHC) | Amino Acid Mutation | Maximum Inhibition Rate (%) (the recombinant protein as shown in SEQ ID NO: 14) | Maximum Inhibition Rate (%) (Staurosporine) |
|---|---|---|---|---|
| LU5162B | 265.433624 | KRAS p.G12D | 99.85 | 100.03 |
| LU11624B | 282.562592 | BRAF G466R | 99.71 | 100.05 |
| LU1235B | 18.748852 | NA | 98.43 | 100.13 |

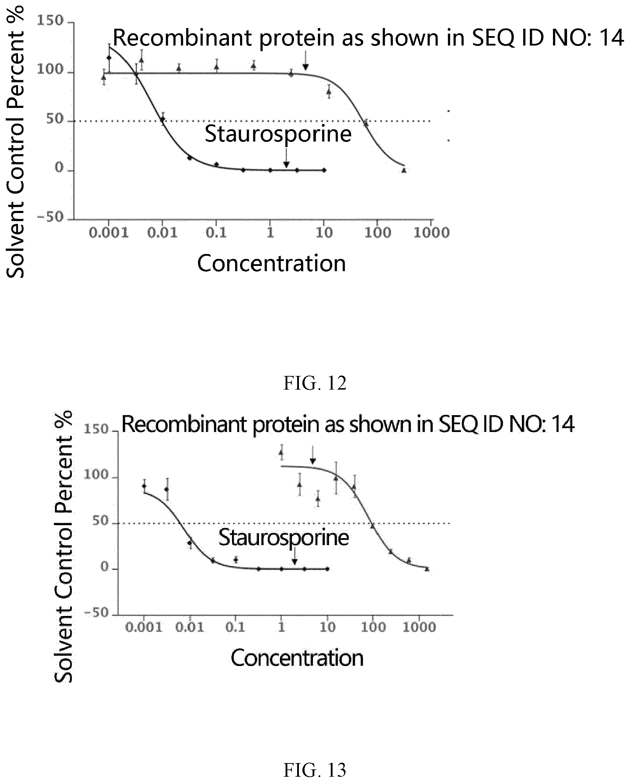

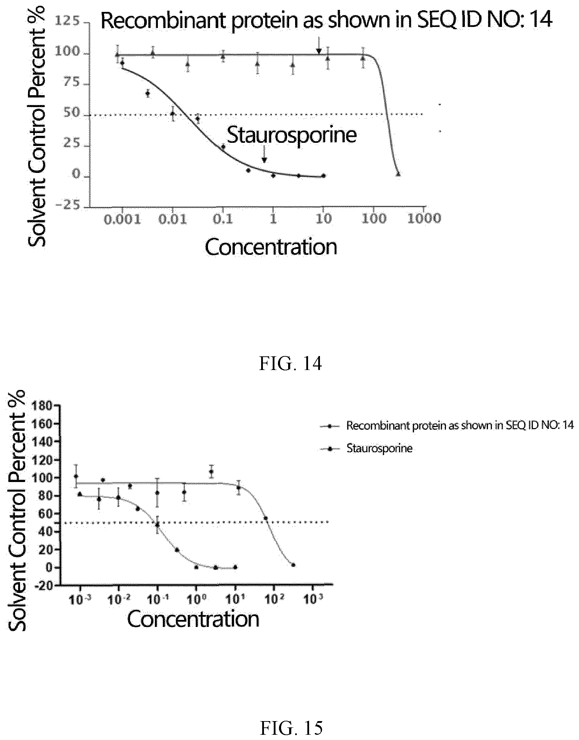

The results are shown in Table 3, and FIG. 12 to FIG. 14: the recombinant protein as shown in SEQ ID NO: 14 has inhibitory effects on the lung cancer cells with EGFR wild-type (LU1235B, FIG. 14), KRAS mutation (LU5162B, FIG. 12) and BRAF mutation (LU11624B, FIG. 13).

### (3) Liver Cancer

**Table 4. Inhibition of Recombinant Protein on Growth of Liver Cancer**

| Organoid Line | EGFR Expression (IHC) | Amino Acid Mutation | Maximum Inhibition Rate (%) (the recombinant protein as shown in SEQ ID NO: 14) | Maximum Inhibition Rate (%) (Staurosporine) |
|---|---|---|---|---|
| LI6677B | 192.205536 | NA | 97.94 | 100.10 |
| LI6669B | 0.33 | NA | 99.98 | 100.07 |

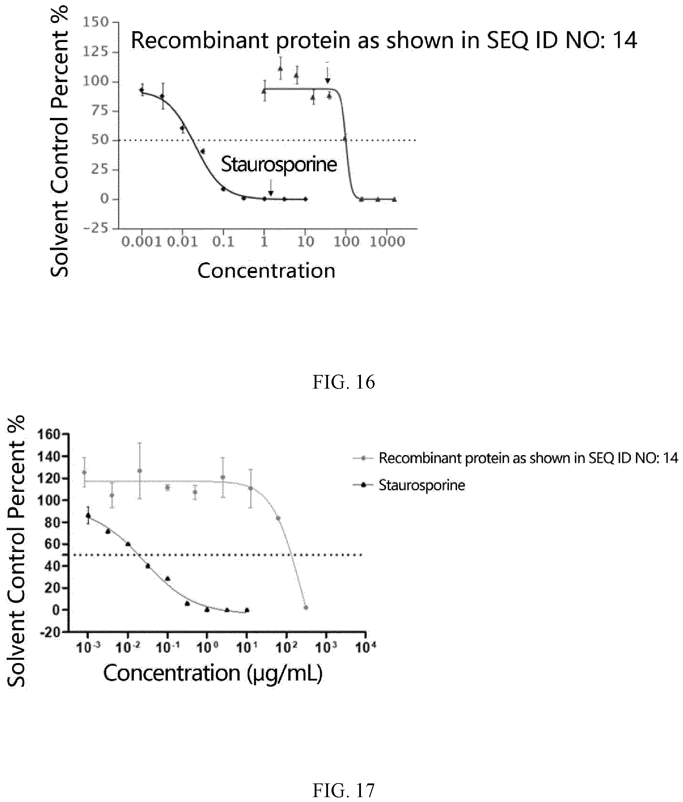

The results are shown in Table 4, and FIG. 15 to FIG. 16: the recombinant protein as shown in SEQ ID NO: 14 has an inhibitory effect on EGFR wild-type liver cells (LI6677B, FIG. 15; LI6669B, FIG. 16).

### (4) Breast Cancer

**Table 5. Inhibition of Recombinant Protein on Growth of Breast Cancer**

| Organoid Line | EGFR Expression (IHC) | Amino Acid Mutation | Maximum Inhibition Rate (%) (the recombinant protein as shown in SEQ ID NO: 14) |
|---|---|---|---|
| BR9457B | 217.465179 | HER 2 | 97.62 |
| BR9466B | NA | HER2 | 97.95 |

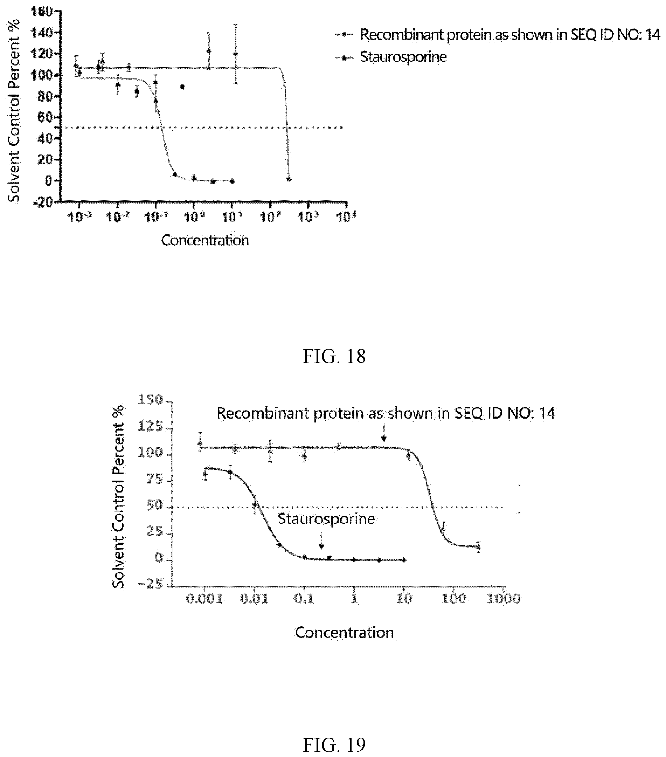

The results are shown in Table 5, and FIG. 17 to FIG. 18: the recombinant protein as shown in SEQ ID NO: 14 has an inhibitory effect on breast cancer cells with HER2 mutation (BR9457B, FIG. 17; BR9466B, FIG. 18).

### (5) Gastric Cancer

**Table 6. Inhibition of Recombinant Protein on Growth of Gastric Cancer**

| Organoid Line | EGFR Expression (IHC) | Amino Acid Mutation | Maximum Inhibition Rate (%) (the recombinant protein as shown in SEQ ID NO: 14) | Maximum Inhibition Rate (%) (Staurosporine) |
|---|---|---|---|---|
| GA6833B | 120.204132 | NA | 87.6 | 100.01 |
| GA2434B | 122.899391 | BRAF | 82.33 | 99.98 |
| | | V600E | | |

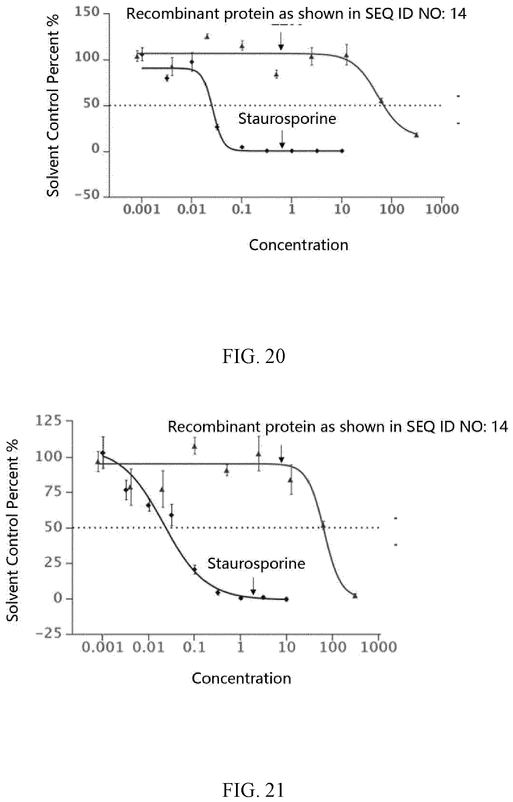

The results are shown in Table 6, and FIG. 19 to FIG. 20: the recombinant protein as shown in SEQ ID NO: 14 has inhibitory effects on gastric cancer cells of EGFR wild-type (GA6833B, FIG. 19) and BRAF mutation (GA2434B, FIG. 20).

### (6) Pancreatic Cancer

**Table 7. Inhibition of Recombinant Protein on Growth of Pancreatic Cancer**

| Organoid Line | EGFR Expression (IHC) | Amino Acid Mutation | Maximum Inhibition Rate (%) (the recombinant protein as shown in SEQ ID NO: 14) | Maximum Inhibition Rate (%) (Staurosporine) |
|---|---|---|---|---|
| PA20078B | 264.937042 | NA | 98.05 | 100.72 |
| PA0787B | 156.790619 | KRAS/BRAF | 97.9 | 100.06 |
| | | G12D/G32_A33dup | | |

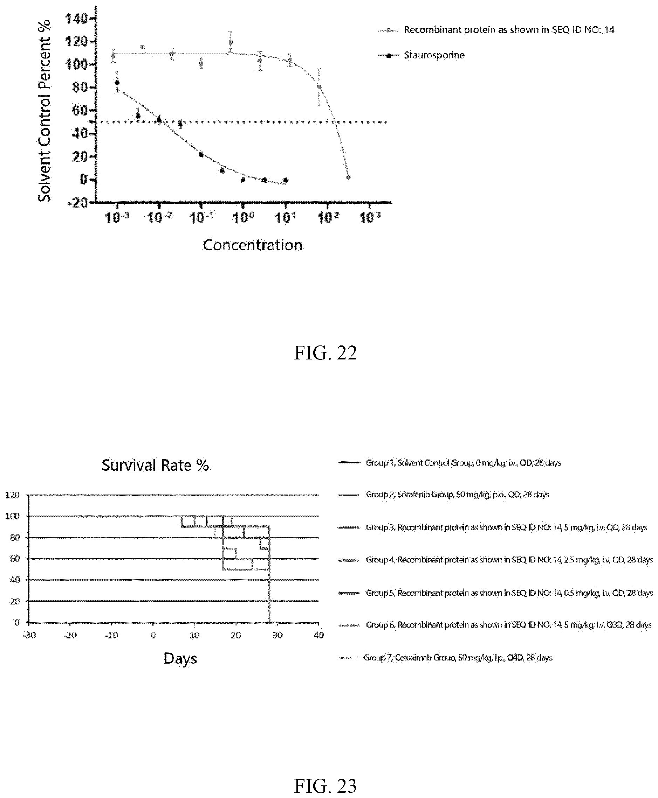

The results are shown in Table 7, and FIG. 21 to FIG. 22: the recombinant protein as shown in SEQ ID NO: 14 has inhibitory effects on pancreatic cancer cells of EGFR wild-type (PA20078B, FIG. 21) and pancreatic cancer cells with the simultaneous mutation of KRAS 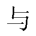 BRAF(PA0787B, FIG. 22).

### Example 10. In Vivo Inhibition of Recombinant Protein on Tumor Growth (PDX Model)

This example performs a pharmacodynamic evaluation of the test product, the recombination protein as shown in SEQ ID NO: 14, in a BALB/c male nude mouse model of orthotopic transplantation of liver cancer LI6669.

### Design of Experiment:

The number of animals in each group, as well as the particular drug delivery route, dose, and regimen are listed in Table 8 below.

**Table 8. Drug Delivery Route, Dose, and Regimen in the LI6669 Amina Model**

| Group | Numb er of Anima ls | Name of Group | Dose (mg/k g) | Administrati on Concentratio n (mg/ml) | Administrati on Volume (µL/g) | Administrati on Mode | Administrati on Cycle |
|---|---|---|---|---|---|---|---|
| 1 | 10 | Solvent (Normal Saline) | - | - | 10 | i.v. | Once a day for 28 days |
| 2 | 10 | Sorafenib | 50 | 5 | 10 | p.o. | Once a day for 28 days |
| 3 | 10 | the recombina nt protein as shown in SEQ ID NO: 14 | 5 | 0.5 | 10 | i.v. | Once a day for 28 days |
| 4 | 10 | the recombina nt protein as shown in SEQ ID NO: 14 | 2.5 | 0.25 | 10 | i.v. | Once a day for 28 days |
| 5 | 10 | the recombina nt protein as shown in SEQ ID NO: 14 | 0.5 | 0.05 | 10 | i.v. | Once a day for 28 days |
| 6 | 10 | the recombina nt protein as shown in SEQ ID NO: 14 | 5 | 0.5 | 10 | i.v. | Once every three days for 28 days |
| 7 | 10 | Cetuxima b | 50 | 5 | 10 | i.p. | Once every four days for 28 days |

Of those, i.p. represents intraperitoneal injection, i.v. represents tail vein injection; and p.o. represents orally intragastrical administration.

The main observation indexes of this experiment are:
Relative tumor proliferation rate, T/C(%), i.e., a percent of the relative tumor volume or tumor weight of the treatment group to the control group at a certain time point. The calculation formula is:
T/C % = TRTV / CRTV × 100% (TRTV: the average RTV of the treatment group; CRTV: the average RTV of the control group; RTV = Vt/V0, wherein V0 is the tumor volume of the animal at the time of grouping, and Vt is the tumor volume of the animal after treatment);
or T/C % = TTW / CTW × 100% (TTW: the average tumor weight at the end of the experiment of the treatment group; and CTW: the average tumor weight at the end of the experiment of the control group).

The calculation formula of the relative tumor inhibition, TGI (%), is:
TGI% = (1-T/C) × 100% (T and C are the relative tumor volume (RTV) or tumor weight (TW) of the treatment group and the control group at a particular time point, respectively).

### Experimental Animals:

| | |
|---|---|
| Species: | Nude Mouse |
| Strain: | BALB/c nude |
| Age in Weeks: | 6-8 weeks (the expected age in weeks of mice at the time of tumor |
| | cell inoculation) |
| Gender: | Male |
| Number of Animals: | A total of 140 mice (70 plus 100% surplus) |
| Supplier: | Jiangsu GemPharmatech Biotechnology Co., Ltd |

Environmental conditions of the animal feeding room:

| | |
|---|---|
| Cage: | Constant temperature, constant humidity, individual ventilated cage polysulfone mouse IVC cage (325 mm × 210 mm × 180 mm), independent air supply system, 10-20 air changes/hour, and IACUC approval number, experiment number, experimental start time, project leader, experiments, animal source, group, and animal number marked on the cage card, etc. |
| Feeding density: | Up to 5 animal per cage |
| Temperature: | 20-26°C |
| Humidity: | 40-70% |
| Light conditions: | Day and night alternate, with light from 7: 00 a.m. to 7: 00 p.m., and darkness from 7: 00 p.m. to 7: 00 a.m. the next day. |
| Bedding: | Corncob beddings sterilized at high pressure, replaced once a week |
| Food supply: | Sustainedly supplied ⁶⁰Co-radiosterilized mouse complete pellet feed, intake ad libitum |
| Water supply: | Drinking tap water (used after high-pressure steam sterilization), uninterrupted water supply with drinking bottle, intake ad libitum |
| Animal Tag: | The experimental animals were marked with ear numbers or tags. |

### Experiment Method:

LI6669 is a HuPrime^{®} orthotopic transplantation model originated from an Asian patient's liver cancer. This model has a certain tendency of tumor collapse.

Tumor tissue was collected from a LI6669 tumor-bearing mouse, a HuPrime^{®} orthotopic transplantation model of liver, and cut to tumor pieces with a diameter of 2-3 mm, which were inoculated into the upper left lobe of liver of a Balb/c nude mouse.

When the average tumor volume of the tumor-bearing mice reached about 50-150 mm³, the mice were randomly grouped by weight according to Table 3 of "3. Design of Experiment". The grouping day was set as Day 0, and the administration began on Day 1. The grouping day was dated as Day 0.

The mice were grouped by weight using StudyDirectorTM (Version 3.1.399.19, Supplier: Studylog System, Inc., S. San Francisco, CA, USA).

"Matched distribution" random grouping method was selected for grouping. This algorithm matched the individual weight measurement of all the selected animals with the average of all the selected animals. First, the paired animals with average close to the average of all the selected animals were selected and assigned to a group so that the average of the group matched (or was as close as possible to) the average of all the selected animals. The final average of measurement of each group would be as close as possible to the final average of measurement of other groups.

### Experiment Observation and Data Collection:

After tumor cell inoculation, route monitoring comprises the tumor growth and the effect of treatment on normal behaviors of animals, specially including the activity, food intake and water consumption, weight gain or loss, eyes, fur of experimental animals, and other abnormal conditions. The clinical symptoms observed during the experiments were all recorded in the original data. After drug administration, the mice were measured twice a week for their weights. Observation was made to record the mouse requiring euthanasia and the corresponding survival time.

The tumor volume would be measured when the mouse was dissected at the end of experiment. The calculation formula of tumor volume: Tumor Volume (mm³) = 1/2 × (a × b²) (wherein a represents a long diameter, and b represents a short diameter). In the experiments, the software StudyDirectorTM (Version 3.1.399.19, Supplier: Studylog System, Inc.) was used to collect data, including measuring the long and short diameters of tumor and the weight of animal. The original data were directly introduced into the software after measurement by balance and vernier caliper. Any change in data would be recorded in this software. All the processes including administration, tumor measurement, and weighing were carried out in a biosafety cabinet or ultra-clean bench.

### Statistical analysis:

To compare the tumor volumes of different treatment groups on a certain day, we first used Bartlett test to verify the hypothesis of homogeneity of variance among all the groups. When the p value of Bartlett test was not less than 0.05, one-way ANOVA would be used to test whether the mean values of all the groups were equal. If the p value of one-way ANOVA was less than 0.05, Tukey HSD test would be used to compare all the groups pairwise, or Dunnett's t-test would be used to compare each treatment group and control group pairwise. When the p value of Bartlett test was less than 0.05, Kruskal Wallis test would be used to test whether the medians of all the groups were equal. When the p value of Kruskal Wallis test was less than 0.05, Conover test would be used to compare all the groups pairwise or compare each treatment group and control group pairwise, and the corresponding p-value correction would be made in accordance with the number of groups in multiple tests.

Moreover, for the purpose of exploratory data analysis, we made pairwise comparison among all the groups at any point in time. Since such comparison only used the tumor volume data of the two groups to be compared at a specific time point, there was no need for multiple test correction. Firstly, we used Bartlett test to verify the hypothesis of homogeneity of variance between the two groups. When the p value of Bartlett test was not less than 0.05, we used Welch's t test to compare whether the mean values of the two groups were equal. When the p value of Bartlett test was less than 0.05, we used Mann Whitney U test to compare whether the medians of the two groups were equal.

All the statistical analyses and graphic plotting are done in R language environment (version 3.3.1). Unless otherwise specified, all tests are two-tailed tests, and it is considered statistically significant if the p value is less than 0.05.

### Experiment Results:

The results are shown in Table 9 and FIG. 23: the group with 5 mg/kg of recombinant protein as shown in SEQ ID NO: 14 (i.v. Q3D) has a longer median survival (greater than 28 days) as compared with the control group (17 days), which is statistically significant (p<0.05). And none of the groups with the recombinant protein as shown in SEQ ID NO: 14 show apparent mouse toxicity. It indicates that the recombinant protein of the present application can effectively prolong the survival of mice with liver cancer.

**Table 9. Median Survivals and P-Values of Mice in Various Treatment Groups and Control Groups in LI6669 Human Liver Cancer Orthotopic Transplantation Tumor Model**

| Group | Median Survival (Days) | *P* value^{b} |
|---|---|---|
| Group 1, Solvent control group | 17 | -- |
| Group 2, Sorafenib 50 mg/kg QD | >28 | 0.275 |
| Group 3, the recombinant protein as shown in SEQ ID NO: 14 5 mg/kg, QD | >28 | 0.119 |
| Group 4, the recombinant protein as shown in | 17 | 0.066 |
| SEQ ID NO: 14 2.5mg/kg, QD | | |
| Group 5, the recombinant protein as shown in SEQ ID NO: 14 0.5mg/kg, QD | >28 | 0.430 |
| Group 6, the recombinant protein as shown in SEQ ID NO: 14 5 mg/kg, Q3D | >28 | 0.032 |
| Group 7, Cetuximab 50 mg/kg, Q4D | 17 | 0.044 |

| | | |
|---|---|---|
| NOTE: a. Mean ± SEM; b. compared with Group 1. | | |

### Example 11. In Vitro Pharmacodynamic Experiment of Recombinant Protein

This experiment takes the inhibitory effect of Erbitux on proliferation of A431 cell line as positive control to investigate the inhibitory effects of the recombinant protein as shown in SEQ ID NO: 14 on multiple cancer cell lines. This experiment utilizes Promega Cell Titer-Glokit to detect the cell viability. This reagent comprises recombinant luciferase and fluorescein, and the luciferase catalyzes the oxidization of a substrate and releases a luminescence signal. The oxidization depends on the ATPs released by the lysis of living cells, and the luminescence signal can indirectly reflect the number of the living cells.

The particular steps were as follows:
1) Target cells were collected and counted, and then inoculated into a 96-well plate;
2) The 96-well plate was placed in a cell incubator at 37 °C, and incubated for about 16-20 hours;
3) A pre-prepared working solution of the sample to be tested and a working solution of the positive control were added into the corresponding wells;
4) The cells were cultured in the cell incubator at 37 °C for additional 72 hours;
5) After incubation, a working solution of Cell TiterGlo testing reagent was added into the corresponding well, which stood at room temperature to stabilize the signal;
6) PHERAStar FSX was used to read the plate.

The results are shown in Table 10, indicating that the recombinant protein as shown in SEQ ID NO: 14 with a concentration of 0.1 mg/mL has certain inhibitory effects on the growth of 5637, HCI-H292, Calu-1, ACHN, AsPC-1, OVCAR8, SK-N-AS, Bel-7402, SK-HEP-1, MDA-MB-468, A431, HEP G2, MDA-MB-453, HLE, A549 and SNU-398.

**Table 10. Inhibitory Effects Recombinant Protein As Shown In SEQ ID NO: 14 On Proliferation of Tumor Cell Lines**

| Name of Cell Line | Tumor Type | EGFR Expression (Measured by flow cytometer) | Relative IC50 (mg/mL) | Absolute IC50 (mg/ml) |
|---|---|---|---|---|
| ACHN | Rental Cancer | 178 | ∼ 0.01920 | 0.02409 |
| 5637 | Bladder cancer | 103 | 0.008885 | 0.009354 |
| AsPC-1 | Pancreatic Cancer | 132 | 0.01628 | 0.02673 |
| NCI-H292 | Lung Adenocarcinoma | 204 | ∼ 0.01883 | 0.01905 |
| SK-N-AS | Neuroblastoma | 50.4 | 0.01075 | 0.1172 |
| OVCAR8 | Ovarian Cancer | 59.6 | 0.01191 | - |
| Calu-1 | Lung Cancer | 131 | 0.00643 | 0.006998 |
| SK-HEP-1 | Liver Cancer | 102 | 0.01441 | 0.01513 |
| HLE | Liver Cancer | 158 | 0.02373 | 0.02779 |
| bel-7402 | Liver Cancer | 116 | 0.01 | 0.01045 |
| MDA-MB-453 | Breast Cancer | - | 0.02212 | 0.02344 |
| MDA-MB-468 | Breast Cancer | - | 0.01288 | 0.01580 |
| A549 | Non-Small Cell Lung Cancer | 42.3 | 0.0515 | 0.08082 |
| A431 | Epithelial Squamous Carcinoma | 802 | 0.01559 | 0.01610 |
| HEP G2 | Liver Cancer | 15 | ∼ 0.01782 | 0.02054 |
| SNU-398 | Liver Cancer | 7.36 | 0.01949 | - |

### Example 12. Pre-Clinical Safety Evaluation Experimental of Recombinant Protein

### (1) In vitro hemolysis experiment of human erythrocytes

This example studies if the recombinant protein as shown in SEQ ID NO: 14 results in lysis or aggregation of human erythrocytes.

Using *in vitro* test tube method, the effect of the test product (*i.e.,* the recombinant protein as shown in SEQ ID NO: 14) on the lysis and aggregation of human erythrocytes. The used concentration of the preparation was 2.5 mg/mL.

Erythrocytes collected from a healthy person was prepared into a 2% (v/v) suspension with sodium chloride injection. Different volumes (2.5 mL ∼ 2.9 mL) of sodium chloride injection and different volumes (0.5 mL ~ 0.1 mL) of the test products were added into glass test tubes containing 2.0 mL of 2% human erythrocyte suspension, respectively. At the same time, 3.0 mL of sodium chloride injection and 3.0 mL of sterilized water for injection were added into glass test tubes containing 2.0 mL of 2% human erythrocyte suspension and taken as negative control and positive controls, respectively. The total volume of each test tube was 5.0 mL. The tubes were incubated in an electrothermal incubator for 3 hours, and observed for the dissolution and aggregation of erythrocytes.

The results show that, in the tube containing the sample and the tube containing the negative control of sodium chloride injection, the erythrocyte settled at the bottom of the tube, and the upper solution was colorless and clear. After shaking, the erythrocytes at the bottom of the tube were uniformly dispersed without hemolysis and coagulation. In the tube containing the positive control of sterilized water for injection, the solution exhibited clear red, no layer separation was observed, no cell residue was observed at the bottom of tube, and complete hemolysis occurred in the tube containing the positive control of sterilized water for injection.

It can be seen that the test product with the concentration of 2.5 mg/mL does not cause hemolysis or aggregation of human erythrocytes *in vitro.*

### (2) Toxicity Test on Bama Miniature Pigs

This example studies the toxic reaction and *in vivo* metabolism in Bama miniature pigs after intravenous infusion of the recombinant protein as shown in SEQ ID NO: 14, and the recovery of toxic reaction 4 weeks after the administration period.

40 Bama miniature pigs (20 pigs/sex) were randomly divided into 4 groups, with 5 pigs/sex in each group. Group 1 was given placebo as the adjuvant control, and Groups 2, 3, and 4 were given 0.5 mg/kg, 1.5 mg/kg, 5.0 mg/kg of the test product (i.e., the recombinant as shown in SEQ ID NO: 14). Administration was carried out by intravenous infusion, once a day for 28 days. The administration volume was 2 mL/kg, and the infusion speed was 30 mL/kg/h. The first 3 animals/sex/group were euthanized after 4 weeks of administration (i.e., D29), and the remaining 2 animals/sex/group were euthanized after 4 weeks of recovery after the last administration (D57).

During the experiment, the animals were examined for clinical observation, weight, temperature, electrocardiogram, ophthalmological examination, blood cell count, coagulation function, blood biochemistry, urine examination, T lymphocyte subgroup, antibody detection, toxicokinetics, organ weight, gross observation and histopathological examination.

The results show that when the Bama miniature pigs were repeatedly intravenously infused with 0.5 mg/kg, 1.5 mg/kg and 5 mg/kg of the test product once a day for 28 days, no death or near-death was found in each group of animals; ; no serious clinical toxic and side effects were found; no toxicological change was found in weight and weight gain, ophthalmological examination, urine analysis and T lymphocyte subgroup in animals in each dose group; no statistically significant change was found in organ weight, organ/brain weight ratio on D29 or D57 in each dose group; and no abnormal change associated with the test product was found in gross observation and microscopic examination at the end of administration period (D29) and at the end of recovery period (D57).

### (3) Test of Safety Pharmacology of Effect on Function of Respiratory System

This example studies the effect of intravenous injection of the recombinant as shown in SEQ ID NO: 14 on the function of respiratory system in rats.

40 SD rats (20 rats/sex, SPF grade) were randomly divided into 4 groups, with 10 rats in each group (5 rats/sex), which were given the adjuvant control and 0.5, 2, 8 mg/kg of the test product (i.e., the recombinant protein as shown in SEQ ID NO: 14) once by intravenous injection. The administration volume was 10 mL/kg. On the day before administration, 10 min after administration, and 24 hours after administration, the animals were placed into a tracing box for detecting the index of respiratory function of the animals.

The results show that, as compared with the adjuvant control group, no statistical difference (P>0.05) and change trend were found in tidal volume, ventilation volume per minute, and respiratory frequency of the animals in the test group on the day before administration, 10 min after administration, and 24 h after administration.

Thus, the single administration of the test product at a dose of 0.5, 2, 8 mg/kg by intravenous injection did not have significant effect on the function of the respiratory system in SD rats.

### (4) Test of Safety Pharmacology of Effect on Function of Central Nervus System

This example studies the effect of intravenous injection of the recombinant as shown in SEQ ID NO: 14 on the function of central nervus system in rats.

40 SD rats (20 rats/sex, SPF grade) were randomly divided into 4 groups, with 10 rats in each group (5 rats/sex), which were given the adjuvant control and 0.5, 2, 8 mg/kg of the test product (i.e., the recombinant protein as shown in SEQ ID NO: 14) once by intravenous injection. The administration volume was 10 mL/kg. The test was carried out by blind method, and the administration information was kept secret from the experimental observer. On the day before administration, 10 min after administration, and 24 hours after administration, the animals were observed by the same experimental using the function combination observation method (FOB) and the results were recorded. The FOB comprises cage observation, hand grasping observation, open environment observation and stimulus reaction observation, and measurement of forelimb grasping force, hind limb opening range, and temperature. Statistical analysis was conducted by subtracting the pre-drug value from the post-drug data.

The results show that, as compared with the adjuvant control group, no abnormality was found in the cage observation, hand grasping observation, open environment observation and stimulus reaction observation, and measurement of forelimb grasping force, hind limb opening range, and temperature of the animals of various test groups 10 minutes and 24 hours after administration.

It can be seen that the single administration of the test product at a dose of 0.5, 2, 8 mg/kg by intravenous injection did not have significant effect on the function of the central nervus system in SD rats.

### (5) Test of Safety Pharmacology of Effect on Function of Cardiovascular System

This example studies the effect of intravenous injection of the recombinant as shown in SEQ ID NO: 14 on the function of cardiovascular system in conscious Bama miniature pigs.

8 Bama miniature pigs (4 pigs/sex, common grade) were used, and a cross-over design was adopted. 0.5 mg/kg and 3 mg/kg of the test product (i.e. the recombinant protein as shown in SEQ ID NO: 14) and an adjuvant control were administered intravenously (the doses of 0.5 mg/kg and 3 mg/kg of the test product were about 8.4 times and 50.6 times of the effective dose, respectively), the administration volume was 2 mL/kg, and the administrate rate was 30 mL/kg/h. Data in various indexes including ECG, blood pressure, and temperature in the animals from at least 2 hours before administration to at least 48 hours after administration were collected, and statistical analysis and evaluation of various index data of ECG, blood pressure, and temperature at a time point within 1.5 hours before administration, and at time points of 0.25 hours (±5 min), 0.5 hours (±5 min), 1 hour (±10 min), 1.5 hours (±10 min), 2 hours (±10 min), 3 hours (±15 min), 4 hours (±20 min), 6 hours (±30 min), 8 hours (±45 min), 12 hours (±45 min), 24 hours (±1 h), 48 hours (±1 h) after administration.

The results show that, as compared with the adjuvant control, administration with 3 mg/kg test product results in that heart rate increased during 0.5 h to 6 h after administration. QTcB interval shortened during 2 h to 4 h. T wave voltage increased during 0.5 h to 6 h.

As compared with the adjuvant control, heat rate-associated change occurred in RR interval, PR interval, and QT interval after administration of 3 mg/kg of the test product.

No statistical difference (P>0.05) was found in other ECG indexes (QRS time limit, QRS voltage, ST segment voltage, Tp-e interval, P wave width), blood pressure indexes (systolic blood pressure, diastolic blood pressure, mean arterial pressure, pulse pressure difference) and temperature of animals with different doses of the test product, and no change trend was found.

It can be seen that intravenous infusion of the test product within the range of pharmacodynamic dose did not have significant effect on the cardiovascular function and temperature of conscious Bama miniature pigs.

In summary, as for the test product, no potential target organ was found within the range of pharmacodynamic dose; no irreversible toxicity was found; and no significant effect was found on the functions of central nervus system, respiratory system, and cardiovascular system.

The aforesaid detailed description are provided in an illustrative and exemplary manner, and are not intended to limit the scope of the appended claims. Various modifications of embodiments currently listed in the present application are apparent for persons skilled in the art, and encompassed within the scope of the appended claims and their equivalences.

## Claims

1. A recombinant protein comprising a spacer peptide and a recombinant *Ganoderma lucidum* immunomodulatory protein mutant (rLZ-8 mutant), wherein said amino acid sequence of the rLZ-8 mutant comprises at least one amino acid mutation as compared with an amino acid sequence as shown in SEQ ID NO: 10.

2. The recombinant protein according to claim 1, wherein said rLZ-8 mutant comprises an amino acid sequence as shown in any one of SEQ ID NOS: 13 and 16-21.

3. The recombinant protein according to any one of claims 1-2, wherein said rLZ-8 mutant comprises an amino acid sequence as shown in SEQ ID NO: 13.

4. The recombinant protein according to any one of claims 1-3, wherein said spacer peptide comprises 1-5 EAs.

5. The recombinant protein according to any one of claims 1-4, wherein said spacer peptide comprises 2 EAs.

6. The recombinant protein according to any one of claims 1-5, wherein said spacer peptide comprises an amino acid sequence as shown in any one of SEQ ID NOS: 8-9.

7. The recombinant protein according to any one of claims 1-6, wherein said spacer peptide comprises an amino acid sequence as shown in SEQ ID NO: 8.

8. The recombinant protein according to any one of claims 1-7, wherein said spacer peptide is located at the N-terminus of said rLZ-8 mutant.

9. The recombinant protein according to any one of claims 1-8, comprising an amino acid sequence as shown in any one of SEQ ID NOS: 14, 15, and 22-33.

10. The recombinant protein according to any one of claims 1-9, comprising an amino acid sequence as shown in SEQ ID NO: 14.

11. An isolated nucleic acid molecule, encoding the recombinant protein of any one of claims 1-10.

12. A vector, comprising the isolated nucleic acid molecule of claim 11.

13. A cell, comprising the isolated nucleic acid molecule of claim 11 and/or the vector of claim 12.

14. The cell according to claim 13, which is a eukaryocyte.

15. The cell according to any one of claims 13-14, which is a yeast cell.

16. A polypeptide, comprising the recombinant protein of any one of claims 1-10.

17. A method of preparing the recombinant protein according to any one of claims 1-10 comprising culturing the cell according to any one of claims 13-15 under conditions that allow to express the recombinant protein according to any one of claims 1-10.

18. A pharmaceutical composition, comprising the recombinant protein according to any one of claims 1-10, the isolated nucleic acid molecule according to claim 11, the vector according to claim 12, the cell according to any one of claims 13-15 and/or the polypeptide according to claim 16, and optionally a pharmaceutically acceptable carrier.

19. A kit, comprising the recombinant protein according to any one of claims 1-10, the isolated nucleic acid molecule according to claim 11, the vector according to claim 12, the cell according to any one of claims 13-15, the polypeptide according to claim 16, and/or the pharmaceutical composition according to claim 18.

20. A drug delivery device, comprising the recombinant protein according to any one of claims 1-10, the isolated nucleic acid molecule according to claim 11, the vector according to claim 12, the cell according to any one of claims 13-15, the polypeptide according to claim 16, and/or the pharmaceutical composition according to claim 18.

21. A method of preventing, alleviating, or treating a tumor, comprising administering a subject in need thereof the recombinant protein according to any one of claims 1-10, the isolated nucleic acid molecule according to claim 11, the vector according to claim 12, the cell according to any one of claims 13-15, the polypeptide according to claim 16, and/or the pharmaceutical composition according to claim 18.

22. The method according to claim 21, wherein said tumor comprises a solid tumor.

23. The method according to any one of claims 21-22, wherein said tumor comprises a tumor having EGFR expression and/or abnormal EGFR expression.

24. The method according to claim 21, wherein said tumor comprises a non-solid tumor.

25. The method according to any one of claims 21-24, wherein said tumor comprises colorectal cancer, lung cancer, liver cancer, breast cancer, gastric cancer, renal cancer, bladder cancer, neuroblastoma, ovarian cancer, epithelial squamous carcinoma, and/or pancreatic cancer.

26. Use of the recombinant protein according to any one of claims 1-10, the isolated nucleic acid molecule according to claim 11, the vector according to claim 12, the cell according to any one of claims 13-15, the polypeptide according to claim 16, and/or the pharmaceutical composition according to claim 18 in preparation of a drug for preventing, alleviating or treating a tumor.

27. The recombinant protein according to any one of claims 1-10, the isolated nucleic acid molecule according to claim 11, the vector according to claim 12, the cell according to any one of claims 13-15, the polypeptide according to claim 16, and/or the pharmaceutical composition according to claim 18 for preventing, alleviating, or treating a tumor.
